(19) Europäisches Patentamt
European Patent Office
Office européen des brevets

(11) Publication number: **0 329 785 B1**

# (12) EUROPEAN PATENT SPECIFICATION

(45) Date of publication of patent specification: **30.11.94**

(51) Int. Cl.⁵: **C07D 501/24**, A61K 31/545

(21) Application number: **87900286.3**

(22) Date of filing: **26.12.86**

(86) International application number:
**PCT/JP86/00655**

(87) International publication number:
**WO 87/03875 (02.07.87 87/14)**

The file contains technical information submitted after the application was filed and not included in this specification

## (54) CEPHALOSPORIN DERIVATIVES.

(30) Priority: **26.12.85 JP 291836/85**
**24.02.86 JP 37449/86**
**02.10.86 JP 233356/86**

(43) Date of publication of application:
**30.08.89 Bulletin 89/35**

(45) Publication of the grant of the patent:
**30.11.94 Bulletin 94/48**

(84) Designated Contracting States:
**AT BE CH DE FR GB IT LI LU NL SE**

(56) References cited:
**DE-A- 3 512 225**
**JP-A- 6 097 983**
**JP-A-59 130 295**
**JP-A-59 172 493**

(73) Proprietor: **Eisai Co., Ltd.**
**6-10, Koishikawa 4-chome**
**Bunkyo-ku**
**Tokyo 112 (JP)**

(72) Inventor: **MACHIDA, Yoshimasa**
**500-81, Shimohirooka**
**Sakura-mura, Niihari-gun**
**Ibaraki 305 (JP)**
Inventor: **KAMIYA, Takashi**
**2-8-3, Matsushiro, Yatabe-machi**
**Tsukuba-gun Ibaraki 305 (JP)**
Inventor: **NEGI, Shigeto**
**14-607, Ohte-machi 14-ban**
**Tsuchiura-shi Ibaraki 300 (JP)**
Inventor: **NAITO, Toshihiko**
**1-10-13, Arakawaokinishi**
**Tsuchiura-shi Ibaraki 300-11 (JP)**
Inventor: **KOMATSU, Yuuki**
**2-23-5, Amakubo, Sakura-mura**
**Niihari-gun Ibaraki 305 (JP)**
Inventor: **NOMOTO, Seiichiro**
**1-134-2, Kariya, Ushiku-machi**
**Inashiki-gun Ibaraki 300-12 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **SUGIYAMA, Isao**
**29-4, Higashi-arai**
**Yatabe-machi**
**Tsukuba-gun Ibaraki 305 (JP)**
Inventor: **YAMAUCHI, Hiroshi**
**500-105, Shimohirooka**
**Sakura-mura**
**Niihari-gun Ibaraki 305 (JP)**

(74) Representative: **Eitle, Werner, Dipl.-Ing. et al**
**Hoffmann, Eitle & Partner,**
**Patent- und Rechtsanwälte,**
**Postfach 81 04 20**
**D-81904 München (DE)**

## Description

### OBJECT OF THE INVENTION

The present invention relates to novel cephalosporin derivatives useful for anti-bacterial agents, or their pharmacologically acceptable salts, and a process for the preparation thereof.

Cephalosporin derivatives having quaternary ammonio group, similar to compounds of the present invention, have been conventionally known from Japanese Patent Application Laid-open Nos. 174387/83; 198490/83; 130295/84; 219292/84; 97983/85; 197693/85, etc. However, a very satisfactory compound has been not provided in respect of a compound effective against both Gram-positive bacteria and Gram-negative bacteria (particularly, Pseud. aeruginosa).

DE-A-35 12 225 concerns 7-[2-(5-amino-1,2,4-thiadiazol-3-yl)-2-(substituted)-iminoacetamido]-3-[3-(quaternary ammonio)-1-propen-1-yl]-3-cephem-4-carboxylates which are used as an antibacterial agent.

EP-A-0 137 440 discloses specifically substituted cephalosporin derivatives for the treatment of bacterial infections. Cephalosporin derivatives of the same type are also revealed by EP-0 111 934.

US-A-4 486 586 includes cephalosporin derivatives which comprise an amino thiazolyl moiety at the 7-position.

The present inventors have found that the compounds of the present invention exhibit excellent anti-bacterial activities against both Gram-positive bacteria and Gram-negative bacteria (Pseud. aeruginosa), and have thus accomplished the present invention.

It is therefore an object of the present invention to provide a novel compound useful for an anti-bacterial agent and a process for the preparation thereof as well as a medicine containing the same.

### CONSTRUCTION OF THE INVENTION

The present invention relates to cephalosporin derivatives represented by the formula:

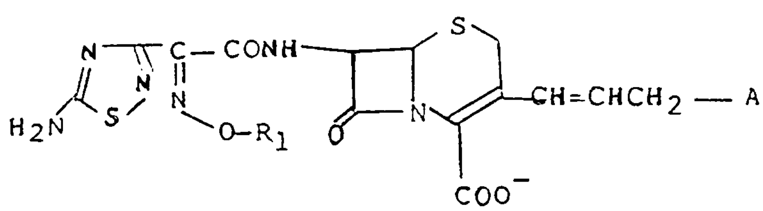

[I]

wherein $R_1$ represents a $C_1$-$C_4$ alkyl group, and A is a group of the following formula:

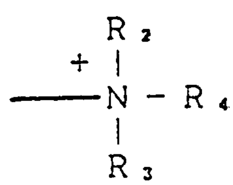

wherein $R_2$ and $R_3$ are the same or different $C_1$-$C_4$ alkyl group, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino, carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent; or its pharmacologically acceptable salt.

The $C_1$-$C_4$ alkyl groups represented by $R_1$ in the above formula (I) include groups such as methyl, ethyl, n-propyl, i-propyl, n-butyl, sec-butyl, t-butyl.

Examples of the $C_1$-$C_4$ alkyl groups defined for A in the formula (I) are those similar to the above examples of the alkyl groups represented by $R_1$.

Examples of the $C_1$-$C_4$ alkanoyl groups represented by $R_2$, $R_3$ and $R_4$ are acetyl and propanoyl.

The 5-membered heterorings in the 5-membered heterocyclic radical represented by $R_4$ include thiazolyl, pyrazolyl, oxadiazolyl, imidazolyl, furyl, iso-thiazolyl, iso-oxazolyl, thiadiazolyl, thienyl, pyrrolyl, tetrazolyl, triazolyl. The substituents on these 5-membered heterorings include amino; carboxyl; $C_1$-$C_4$ alkyl groups such as methyl, ethyl, n-propyl, i-propyl, n-butyl, sec-butyl, t-butyl; carboxy $C_1$-$C_4$ alkyl groups such as carboxymethyl, carboxyethyl; carbamoyl; hydroxyl; $C_1$-$C_4$ alkyl substituted amino groups such as dimethylamino and diethylamino; and hydroxy $C_1$-$C_4$ alkyl groups such as hydroxymethyl and hydroxyethyl.

Illustrative of the groups represented by A are:

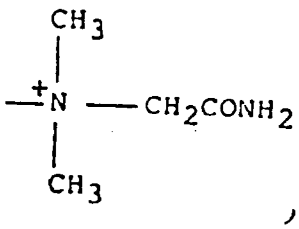 ,
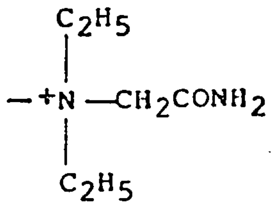 ,
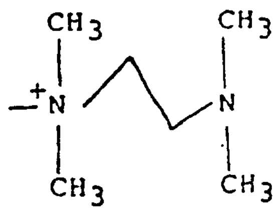 ,

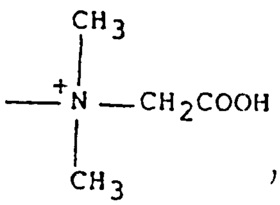 ,
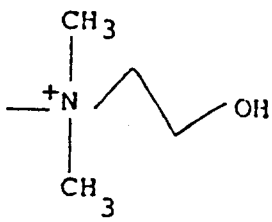 ,
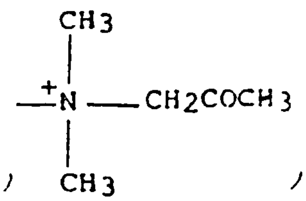 ,

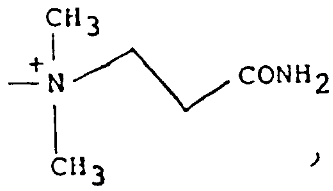,
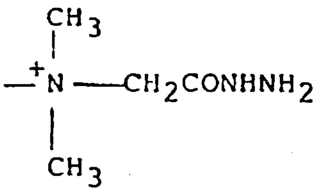,
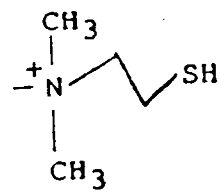,

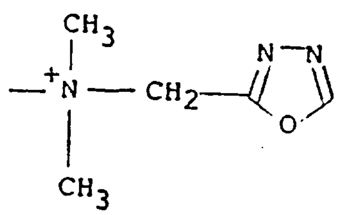,
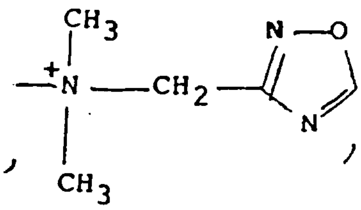,

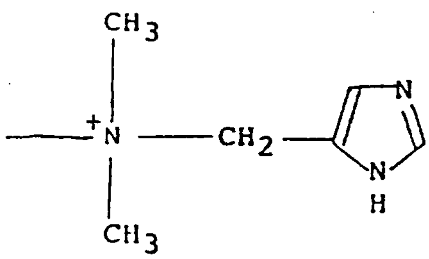,
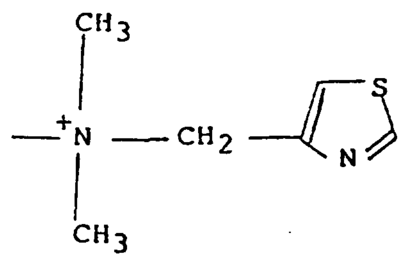,

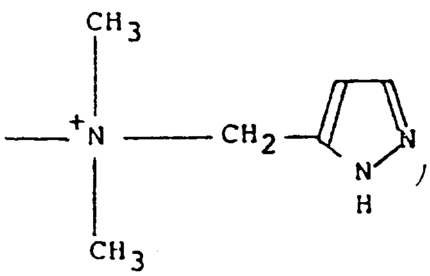,
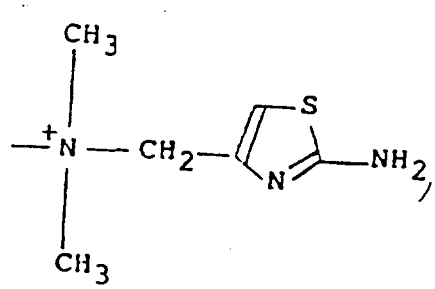,

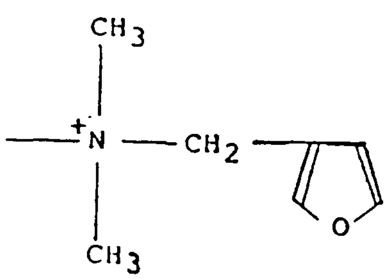,

The pharmacologically acceptable salts of the compounds represented by the formula (I) include medicinally acceptable salts, for example, alkali metal salts such as sodium, potassium salts, etc.; alkaline earth metal salts such as calcium, magnesium salts, etc.; inorganic acid salts such as hydrochlorides, hydrobromides, hydroiodides, sulfates, carbonates, bicarbonates, etc.; organic caroxylic salts such as acetates, maleates, lactates, tartrates, etc.; organic sulfonates such as methane sulfonates, benzene sulfonates, toluene sulfonates, etc.; salts of amino acids such as salts of arginine, lysine, serine, aspartic acid and glutamic acid, etc.; and salts of amine such as salts of trimethylamine, triethylamine, pyridine, procaine, picoline, dicyclohexylamine, N,N'-dibenzylethylene diamine, N-methylglucamine, diethanolamine, triethanolamine, tris-(hydroxymethylamine) methan and phenethylbenzylamine, etc.

For steric configuration of the following moiety of the compound represented by the formula (I) according to the present invention, there are syn-isomer (Z) and anti-isomer (E).

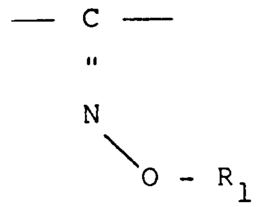

The present invention includes both of such isomers, but the syn isomers are preferred from the viewpoint of antibacterial activities.

The compounds according to the present invention may be prepared in accordance with the following proceses.

The compound of the formula (I) or the pharmacologically acceptable salt thereof can be provided by reacting a compound represented by the formula:

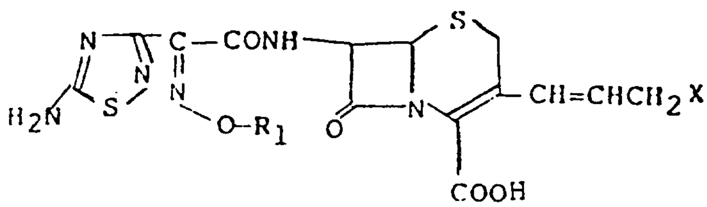

(II)

wherein $R_1$ is a $C_1$-$C_4$ alkyl group and X is halogen atom, a compound resulting from the protection of the amino and/or carboxyl group(s) of the compound by a protective group, or a salt of one of these compounds, with a compound or its salt represented by the formula:

A' (III)

wherein A' is

a group of the following formula:

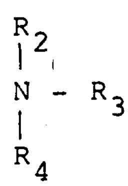

where $R_2$ and $R_3$ are the same or different $C_1$-$C_4$ alkyl groups, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino, carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent; or its pharmacologically acceptable salt, and optionally removing the protective group.

The halogen atoms represented by X in the above formula (II) include iodine, bromine and chlorine atoms. The group A' is a tertiary amine corresponding to the group A in the above formula (I).

The reaction may be carried out at a temperature in a range of -10 °C to 60 °C, preferably 0 °C to 40 °C. Preferable reaction solvents are anhydrous organic solvents. The organic solvents which may be used include lower alkylnitrile such as acetonitrile and propyonitrile; halogenated lower alkyl groups such as chloromethane, dichloromethane and chloroform; ethers such as tetrahydrofurane, dioxane and ethylether; amides such as N,N-dimethylformamide; esters such as ethyl acetate; ketones such as acetone; and hydrocarbones such as benzene, or the mixtures of these solvents.

The removal of the protective group may be carried out in a usual manner, for example, by hydrolysis or reduction and the like, depending upon the type of the protective group used.

For the protective groups for the amino and carboxyl groups in the salts of the compounds of the formulae (II) and (III) and in the compounds of the formula (II), any groups usually employed can be used unless they will prevent the aforesaid reaction.

Illustrative of the protective groups for the amino groups are, for example, formyl, acetyl, chloroacetyl, dichloroacetyl, t-butoxycarbonyl, benzyloxycarbonyl, trityl, p-methoxybenzyl, and diphenylmethyl groups. Illustrative of the protective groups for the carboxyl groups are p-methoxybenzyl, p-nitrobenzyl, t-butyl, methyl, 2,2,2-trichloroethyl, diphenylmethyl and pivaloyloxymethyl groups. It is convenient to use a silylate agent such as bis-(trimethylsilyl) acetoamide, N-methyl-N-(trimethylsilyl) acetoamide, and N-methyl-N-trimethylsilyl-trifluoroacetoamide, because they can concurrently protect both the amino and carboxyl groups.

The salts of the compounds represented by the formulae (II) and (III) can be selected from the group consisting of alkali metal salts such as sodium and potassium salts; alkaline earth metal salts such as calcium and magnesium salts; ammonium salts; inorganic acid salts such as hydrochlorides, hydrobromides, sulfates, carbonates, hydroiodides and bicarbonates; organic carboxylic acid salts such as acetates, trifluoroacetates, maleates, lactate and tartrates; organic sulfonates such as methane sulfonates, benzene sulfonates and toluene sulfonates; salts of amine such as salts of trimethylamine, triethylamine, pyridine, procaine, picoline, dicyclohexylamine, N,N'-dibenzylethylene diamine, N-methylglucamine, diethanolamine, triethanolamine, tris-(hydroxymethylamine)methane and phenethylbenzylamine; and salts of amino acids such as salts of arginine, aspartic acid, lysine, glutamic acid and serine.

The compounds according to the present invention have strong anti-bacterial activities against Gram-negative bacteria and Gram-positive bacteria and are useful as anti-bacterial agents.

Any of the following compounds according to the present invention had an acute toxicity value [$LD_{50}$ -(intravenous injection into mice)] of 3,000 mg/kg or more:

7β-[2-(5-Amino-1,2,4-thiadiazol-3-yl)-(Z)-2-methoxyiminoacetamido]-3-[(E)-3-(carbamoylmethyldimethylammonio)-1-propene-1-yl]-3-cephem-4-carboxylate;

7β-[2-(5-Amino-1,2,4-thiadiazol-3-yl)-(Z)-2-methoxyiminoacetamido]-3-[(E)-3-[(1,3,4-oxadiazol-2-yl) methyldimethylammonio]-1-propene-1-yl]-3-cephem-4-carboxylate.

In using the compounds according to the present invention as injections, they may be intraveneously or intramuscularly administered in an amount of 100 mg to 10 g divided 1 to 4 times per day. It should be noted that the dosages may be increased or decreased depending upon the age and condition of a patient.

The injections can be produced in a usual manner. For example, the compound according to the present invention may be dissolved in distilled water in the presence of an isotonic agent, a dissolving assistant or the like as required, thereby providing an injection. Alternatively, the compound may be charged into a vial or the like in a form of a powder to form an injection of a type which may be dissolved when it is to be used. The injection of this type may be used by dissolving it into a medium such as an injecting distilled-water, a physiological saline solution, a glucose injecting liquid, an amino acid carrier liquid when it is to be administered.

The present invention will now be described in more detail by way of experiments and examples.

Experiment 1 (Synthesis of source compound)

p-Methoxybenzyl 7β-[2-(5-tritylamino-1,2,4-thiadiazol-3-yl)-(Z)-2-methoxyiminoacetamido]-3-chloromethyl-3-cephem-4-carboxylate

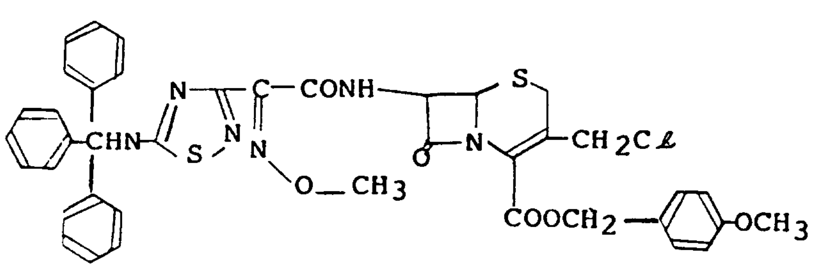

100 G of 2-(5-tritylamino-1,2,4-thiadiazol-3-yl)-(Z)-2-methoxyimino acetic acid were dissolved in 1ℓ of dimethylformamide, and to which 121.7 g of p-methoxybenzyl 7β-amino-3-chloromethyl-3-cephem-4-carboxylate p-toluene sulfonate, 22.77 g of triethylamine and 139.6 g of N-ethyl-N'-3-dimethylaminopropyl carbodiimide hydrochloride were added under ice-cooling, and the whole was stirred at the same temperature for 8 hours. After addition of 4ℓ of ethyl acetate to the reaction solution, it was washed with water, a saturated brine, a saturated aqueous sodium hydrogen carbonate, a saturated brine, 1N hydrochloric acid and a saturated brine, in order. Anhydrous magnesium sulfate was added to the product to dry the same. Thereafter, the solvent was distilled off under vaccum. The residue was purified with a silica gel column chromatography (elution: chloroform) to yield 115 g of the objective compound.

Experiment 2 (Synthesis of source compound)

p-Methoxybenzyl 7β-[2-(5-tritylamino-1,2,4-thiadiazol-3-yl)-(Z)-2-methoxyiminoacetamido]-3-iodomethyl-3-cephem-4-carboxylate

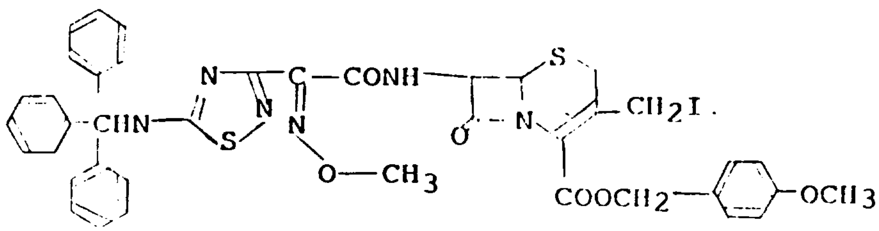

60 G of the compound obtained in the Experiment 1 were dissolved in 1.2ℓ of methylethylketone, and 56.5 g of methyliodide were added thereto, and the whole was stirred at the room temperature for one hour. The solvent was distilled off and the residue was dissolved in ethyl acetate, followed by washing with water, a saturated aqueous sodium thiosulfate solution and a saturated brine in order and it was dried with adding anhydrous magnesium sulfate. The resulting product was concentrated under reduced pressure. n-Hexane was added to the concentrate to collect the resulting precipitates by filtration, and 61.0 g of the objective compound were obtained.

Experiment 3 (Synthesis of source compound)

p-Methoxybenzyl-7β-[2-(5-tritylamino-1,2,4-thiadiazol-3-yl)-(Z)-2-methoxyiminoacetamido]-3-triphenylsulfonyomethyl-3-cephem-4-carboxylate iodide

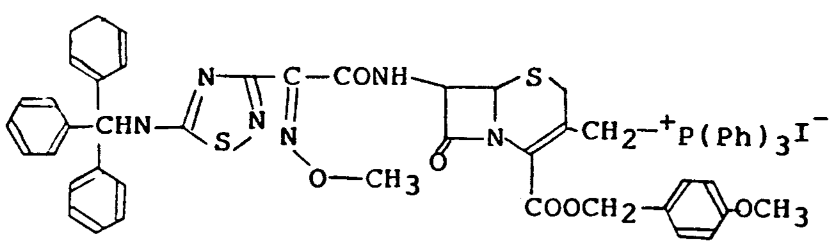

9.54 G of the compound obtained in the Experiment 2 were dissolved in 190 mℓ of benzene, and 5.64 g of triphenylphosphine were added thereto. The mixture was stirred at the room temperature for one hour. To this reaction solution were added dropwise 100 mℓ of n-hexane, and the resulting precipitate was collected by filtration. The precipitate was then washed with a liquid mixture of benzene-n-hexane (1:1) and n-hexane to yield 11.30 g of the objective compound.

Experiment 4 (Synthesis of source compound)

p-Methoxybenzyl 7β-[2-(5-tritylamino-1,2,4-thiadiazol-3-yl)-(Z)-2-methoxyiminoacetamido]-3-[(Z)-3-chloro-1-propen-1-yl]-3-cephem-4-carboxylate

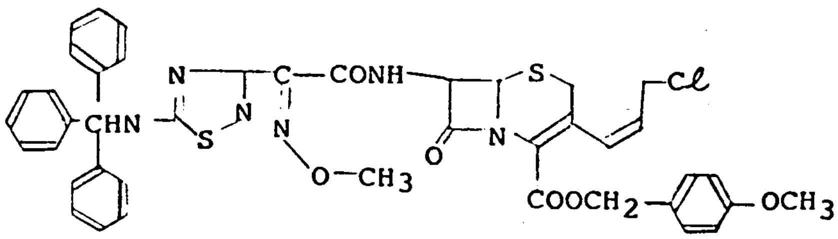

30.0 G of the compound obtained in the Experiment 3 were dissolved in a liquid mixture of 250 mℓ of chloroform and 300 mℓ of ethyl acetate, and the mixture was stirred under ice-cooling with adding 78.3 mℓ of an aqueous solution of 1N sodium hydroxide. The organic layer was separated to collect, and washed with a saturated brine, followed by drying with addition of anhydrous magnesium sulfate. To this organic layer were added 6.15 g of chloroacetaldehyde solution in 10 mℓ of chloroform. The mixture was stirred at the room temperature for one hour. The reaction solution was concentrated, and 300 mℓ of ethyl acetate were added thereto. The resulting precipitate was filtered off. The filtrate was concentrated to yield 29.3 g of crude product. This product was purified by silica gel column chromatography (elution: chloroform) to yield 7.73 g of the objective compound.

Experiment 5 (synthesis of source compound)

p-Methoxybenzyl 7β-[2-(5-tritylamino-1,2,4-thiadiazol-3-yl)-(Z)-2-methoxyiminoacetamido]-3-[(E)-iodo-1-propen-1-yl]-3-chephem-4-carboxylate

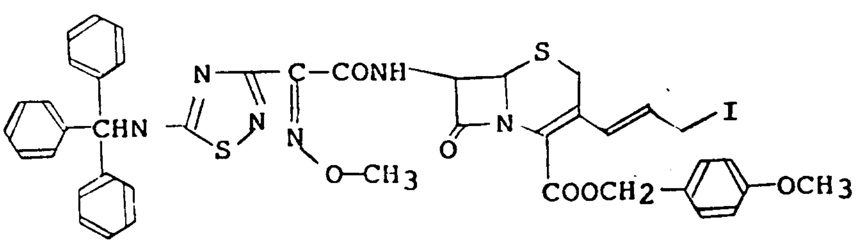

7.97 G of the compound obtained in the Experiment 4 were dissolved in 167 mℓ of acetone, and 4.37 g of sodium iodide were added thereto. The mixture was stirred at the room temperature for one hour. The solvent was distilled off. The residue was dissolved in 150 mℓ of ethyl acetate, and the solution was washed with a saturated aqueous sodium thiosulfate solution and then a saturated brine, followed by drying with addition of anhydrous magnesium sulfate. The dried residue was concentrated under a reduced pressure. The residue was solidified by addition of n-hexane and petroleum ether, and collected by filtration to yield 8.42 g of the objective compound.

Experiment 6 (Synthesis of source compound)

p-Methoxybenzyl 7β-[2-(5-tritylamino-1,2,4-thiadiazol-3-yl)-(Z)-2-ethoxyiminoacetamido]-3-chloromethyl-3-cephem-4-carboxylate

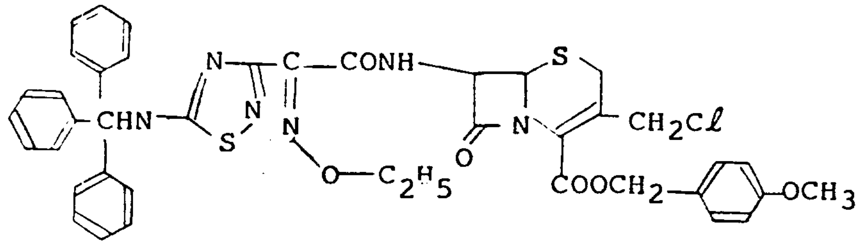

In the same manner as in the Experiment 1, 21.5 g of 2-(5-tritylamino-1,2,4-thiadiazol-3-yl)-(Z)-2-ethoxyimino acetic acid were reacted with 19.8 g of p-toluenesulfonate of p-methoxybenzyl 7β-amino-3-chloromethyl-3-cephem-4-carboxylic acid to yield 13.4 g of the objective compound.

Experiment 7 (Synthesis of source compound)

p-Methoxybenzyl 7β-[2-(5-tritylamino-1,2,4-thiadiazol-3-yl)-(Z)-2-ethoxyiminoacetamido]-3-(3-chloro-1-propen-1-yl)-3-cephem-4-carboxylate

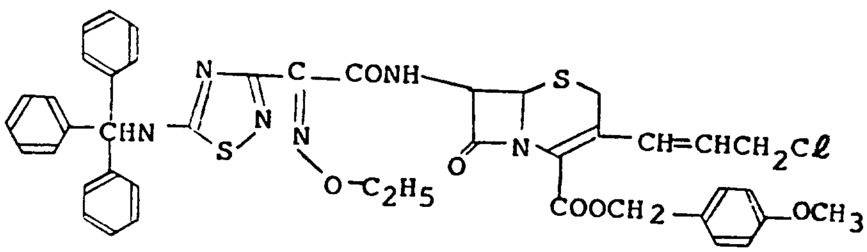

13 G of the compound obtained in the Experiment 6, 3 g of sodium iodide and 8.5 g of triphenylphosphine were suspended in 80 mℓ of acetone and stirred. Aftet two hours, 300 mℓ of isopropyl ether were added, and 18.8 g of the resulting precipitate were collected by filtration. The precipitate was suspended in 100 mℓ of chloroform, and then 46 mℓ of 1N aqueous solution of sodium hydroxide were added thereto. The organic layer was collected and dried with adding anhydrous magnesium sulfate thereto. Then, 3.6 g of chloroacetaldehyde were added, and the mixture was stirred for one hour. The reaction solution was concentrated under reduced pressure, and 300 mℓ of ethyl acetate were added thereto. The resulting precipitate was removed by filtration. The filtrate was concentrated and purified with a silica gel column chromatography to yield 3.46 g of the objective compound.

Experiment 8 (synthesis of source compound)

p-Methoxybenzyl 7β-[2-(5-tritylamino-1,2,4-thiadiazol-3-yl)-(Z)-2-ethoxyiminoacetamido-3]-(3-iodo-1-propen-1-yl)-3-cephem-4-carboxylate

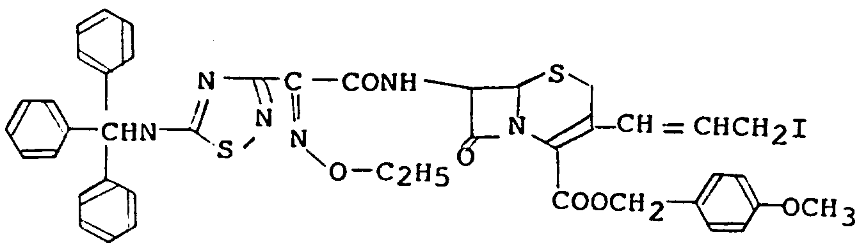

In the same manner as in the Experiment 5, 1.7 g of the compound of the Experiment 7 were reacted with 600 mg of sodium iodide to obtain 1.1 g of the objective compound.

Example 1

7β-[2-(5-Amino-1,2,4-thiadiazol-3-yl)-(Z)-2-methoxyiminoacetamido]-3-[(E)-3-(carbamoylmethyldimethylammonio)-1-propen-1-yl)-3-cephem-4-carboxylate

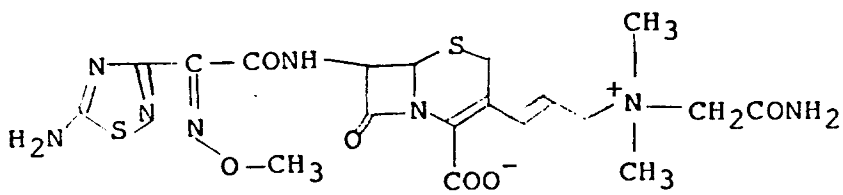

600 Mg of the compound obtained in Experiment 5 were suspended in 60 mℓ of ethyl ether, and then 87.3 mg of N,N-dimethylglycineamide were added thereto, followed by stirring at the room temperature for 10 minutes. To this solution, were added dropwise 43 mℓ of ethyl acetate over 30 minutes. After stirring the mixture for 12 hours, 20 mℓ of ethyl ether were added thereto, and the resulting precipitate was recovered by filtration. The precipitate was dissolved in 1 mℓ of tetrahydrofuran, and then 10 mℓ of ethyl acetate were added to the solution. The resulting precipitate was recovered by filtration to yield 279 mg of a yellow powder.

This product was added to a liquid mixture of 3.32 mℓ of trifluoroacetic acid and 1.75 mℓ anisole, followed by stirring under ice-cooling for 3 hours. The reaction solution was added to 50 mℓ of ethyl ether and 50 mℓ of isopropyl ether. The resulting precipitate was recovered by filtration and washed with ethyl ether. The precipitate was added to 1.8 mℓ of water, and pH of the solution was adjusted to 6.0 by means of sodium acetate. Insoluble matters are removed by filtration. The filtrate was purified by a reverse-phase silica gel column chromatography to yield 51 mg of the objective compound.

Example 2

7β-[2-(5-Amino-1,2,4-thiadiazol-3-yl)-(Z)-2-methoxyiminoacetamido]-3-[(E)-3-carboxymethyldimethyl-ammonio)-1-propen-1-yl]-3-cephem-4-carboxylate

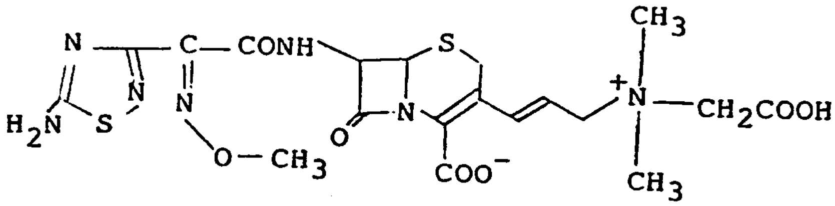

800 Mg of the compound obtained in Experiment 5 were reacted with 117.5 mg of N,N-dimethylglycine to yield 12 mg of the objective compound.

Example 3

7β-[2-(5-Amino-1,2,4-thiadiazol-3-yl)-(Z)-2-methoxyiminoacetamido]-3-[(E)-3-(carbamoylmethyldiethyl-ammonio)-1-propen-1-yl]-3-cephem-4-carboxylate

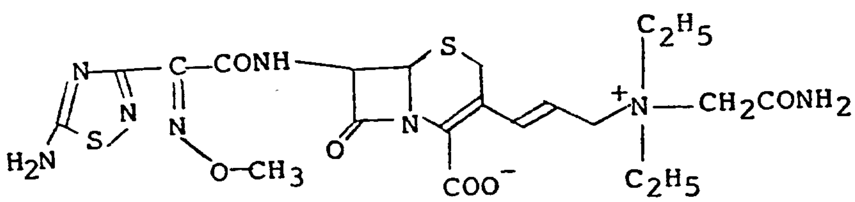

500 Mg of the compound obtained in Experiment 5 were reacted with 93 mg of N,N-diethyl-glycineamide to yield 6 mg of the objective compound.

Example 4

7β-[2-(5-Amino-1,2,4-thiadiazol-3-yl)-(Z)-2-methoxyiminoacetamido]-3-[(E)-3-(2-dimethylaminoethyldimethyl-ammonio)-1-propen-1-yl]-3-cephem-4-carboxylate

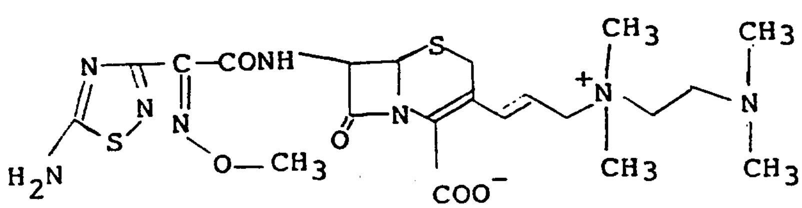

800 Mg of the compound obtained in Experiment 5 were reacted with 172 μl of 1,2-bis(dimethylamino)-ethane to yield 14 mg of the objective compound.

Example 5

7β-[2-(5-Amino-1,2,4-thiadiazol-3-yl)-(Z)-2-methoxyiminoacetamido]-3-[(E)-(2-mercaptoethyldimethylammonio)-1-propen-1-yl]-3-cephem-4-carboxylate

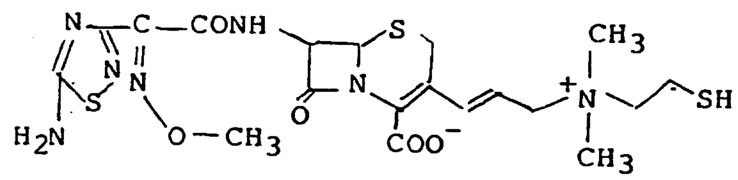

500 Mg of the compound obtained in Experiment 5 were reacted with 200 mg of 2-dimethylaminoethanethiol to yield 2.5 mg of the objective compound.

Example 6

7β-[2-(5-Amino-1,2,4-thiadiazol-3-yl)-(Z)-2-ethoxyiminoacetamido]-3-[(E)-3-(carbamoylmethyldimethylammonio)-1-propen-1-yl]-3-cephem-4-carboxylate

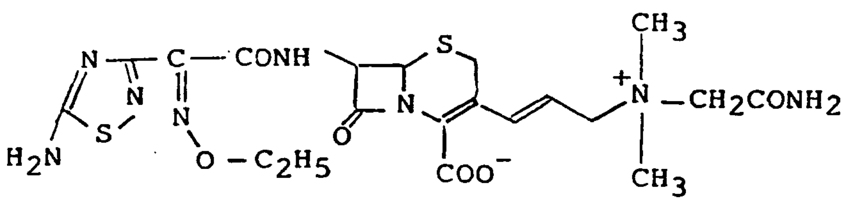

1.0 G of the compound obtained in Experiment 8 were reacted with 230 mg of N,N-dimethylglycineamide to yield the objective compound.

Example 7

7β-[2-(5-Amino-1,2,4-thiadiazol-3-yl)-(Z)-2-methoxyiminoacetamido]-3-[(E)-3-(2-oxopropyldimethylammonio)-1-propen-1-yl]-3-cephem-4-carboxylate

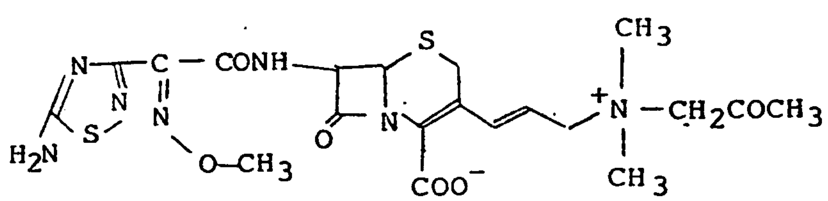

500 Mg of the compound obtained in Experiment 5 were reacted with 100 mg of N,N-dimethylaminoacetone to yield 36 mg of the objective compound.

Example 8

7β-[2-(5-Amino-1,2,4-thiadiazol-3-yl)-(Z)-2-methoxyiminoacetamido]-3-[(E)-3-(2-hydroxyethyldimethylammonio)-1-propen-1-yl]-3-cephem-4-carboxylate

(Compound A)

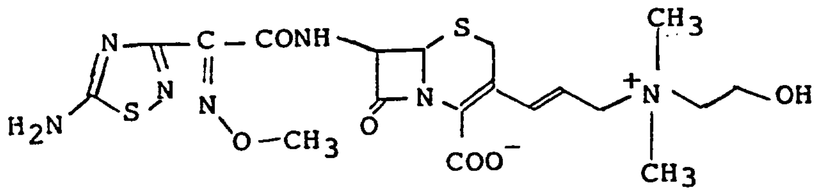

7β-[2-(5-Amino-1,2,4-thiadiazol-3-yl)-(Z)-2-methoxyiminoacetamido]-3-[(Z)-3-(2-hydroxyethyldimethylammonio)-1-propen-1-yl]-3-cephem-4-carboxylate

(Compound B)

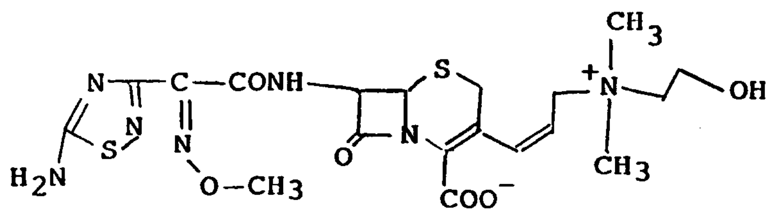

500 Mg of the compound obtained in Experiment 4, 142 mg of sodium iodide and 10 mℓ of 2-butanone were stirred at room temperature for one hour. The solvent was distilled off. The residue was dissolved in ethyl acetate. The resulting solution was washed with 10% aqueous solution of sodium thiosulfate and saturated brine, followed by drying with an addition of anhydrous magnesium sulfate. The solvent was distilled off, and the residue was dissolved in a liquid mixture of 30 mℓ of ethyl acetate and 10 mℓ of ethyl ether. To this solution were added 112 mg of N,N-dimethylaminoethanol, and the solution was stirred at the room temperature for one hour and 30 minutes. 20 Mℓ of ethyl ether were added to the solution. The resulting precipitate was recovered by filtration. This precipitate was washed with isopropyl ether to yield 370 mg of a yellow powder.

This yellow powder was added, under ice-cooling, to a liquid mixture of 0.8 mℓ of anisole and 8 mℓ of trifluoroacetic acid, and the whole was stirred at the same temperature for one hour. To the reaction solution were added 20 mℓ of isopropyl ether, and the resulting precipitate was recovered by filtration. To this precipitate was added 30% aqueous methanol. Insoluble matters were removed by filtration. The pH of the filtrate was adjusted to 5 by an addition of sodium acetate. The solvent was distilled off. The residue was purified by a reversed-phase silica gel column chromatography (elution: 5% aqueous methanol) to yield 25 mg of the objective compound A and 17 mg of the compound B.

Example 9

7β-[2-(5-Amino-1,2,4-thiadiazol-3-yl)-(Z)-2-methoxyiminoacetamido]-3-[(Z)-3-(carbamoylmethyldimethyl-ammonio)-1-propen-1-yl]-3-cephem-4-carboxylate

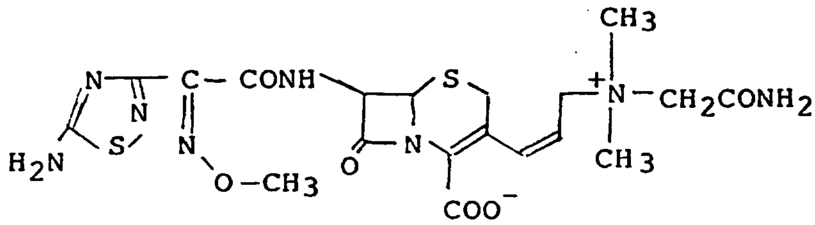

1.031 G of the compound obtained in Experiment 3 were dissolved in a liquid mixture of 20 mℓ of ethyl acetate and 6 mℓ of chloroform, and then 20 mℓ of 10% aqueous solution of potassium carbonate were added thereto, followed by shaking. The organic layer was taken out and dried with an addition of anhydrous potassium carbonate, followed by washing with 6 mℓ of ethyl acetate. A solution of 85 mℓ of chloroacetaldehyde in 0.25 mℓ of ethyl acetate was added thereto under ice-cooling, followed by stirring for 4 hours. Thereafter, 137 mg of N,N-dimethylglycinamide and a solution of 50 mg of sodium iodide in 1 mℓ of acetone were added thereto, followed by stirring at the room temperature overnight. The deposits were recovered by filtration and dried. The deposits were dissolved in 1.4 mℓ of anisole and the resulting solution was stirred for one hour under ice-cooling with an addition of 1.9 mℓ of trifluoroacetic acid. To the reaction solution were added 100 mℓ of a liquid mixture of isopropyl ether - ethyl ether (1:1), and the resulting precipitates were recovered by filtration. The precipitates were washed with ethyl ether, and dried to obtain 20 mg of solid. This solid was dissolved in a liquid mixture of 1.5 mℓ of water - 1.0 mℓ of an aqueous saturated solution of acetate, and the insoluble matters were removed by filtration. The filtrate was purified by a reversed-phase silica gel column chromatography (elution: a gradient of water-6% aqueous methanol) to yield 5 mg of the objective compound.

Example 10

7β-[2-(5-Amino-1,2,4-thiadiazol-3-yl)-(Z)-2-methoxyiminoacetamido]-3-[(E)-3-[(1,3,4-oxadiazol-2-yl)methyldi-methylammonio]-1-propen-1-yl]-3-cephem-4-carboxylate

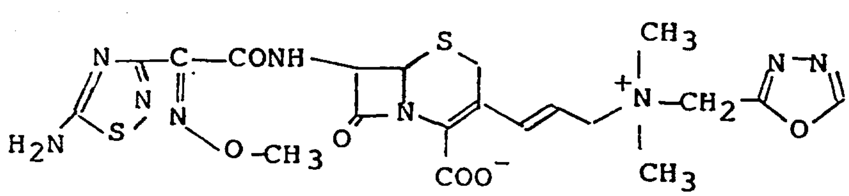

500 Mg of the compound obtained in Experiment 5 were suspended in 10 mℓ of ethyl ether, followed by adding 120 mg of 2-dimethylaminomethyl-1,3,4-oxadiazol. The mixture was stirred at the room temperature for one hour. To this reaction solution were added 50 mℓ of ethyl ether. The resulting precipitate was recovered by filtration to yield 510 mg of a yellow powder.

This yellow powder was added to a liquid mixture of 2.5 mℓ of trifluoroacetic acid and 2.5 mℓ of anisole, followed by stirring under ice-cooling for 3 hours. To this reaction solution, 50 mℓ of isopropyl ether were added, and the resulting precipitate was recovered by filtration. This precipitate was suspended in 20 mℓ of water. The pH of the suspension was adjusted to 7 - 8 by an addition of sodium hydrogencarbonate. The insoluble matters were removed by filtration. The filtrate was purified by a reversed-phase silica gel chromatography to yield 13 mg of the objective compound.

Example 11

7β-[2-(5-Amino-1,2,4-thiadiazol-3-yl)-(Z)-2-methoxyiminoacetamido]-3-[(E)-3-[(1,2,4-oxadiazol-3-yl)-methyldimethylammonio]-1-propen-1-yl]-3-cephem-4-carboxylate

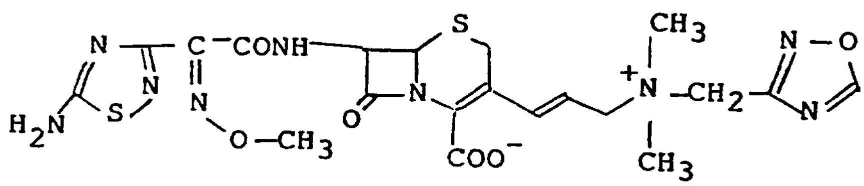

500 Mg of the compound obtained in Experiment 5 were suspended in 10 mℓ of ethyl ether, and then 120 mg of 3-dimethylaminomethyl-1,2,4-oxadiazol were added thereto, followed by stirring at the room temperature for one hour. To this reaction solution were added 50 mℓ of ethyl ether. The resulting precipitate was recovered by filtration to yield 480 mg of a yellow powder.

This yellow powder was added to a liquid mixture of 2.5 mℓ of trifluoroacetic acid and 2.5 mℓ of anisole, followed by stirring under ice-cooling for 3 hours. To this reaction solution were added 50 mℓ of isopropyl ether, and the resulting precipitate was recovered by filtration. This precipitate was suspended in 2 mℓ of water. The pH of the suspension was adjusted to 7 - 8 by an addition of sodium hydrogencarbonate. The insoluble matters were removed by filtration. The filtrate was purified by a reversed-phase silica gel chromatography to yield 34 mg of the objective compound.

Example 12

7β-[2-(5-Amino-1,2,4-thiadiazol-3-yl)-(Z)-2-methoxyiminoacetamido]-3-[(E)-3-[(imidazol-4-yl)-methyldimethylammonio]-1-propen-1-yl]-3-cephem-4-carboxylate

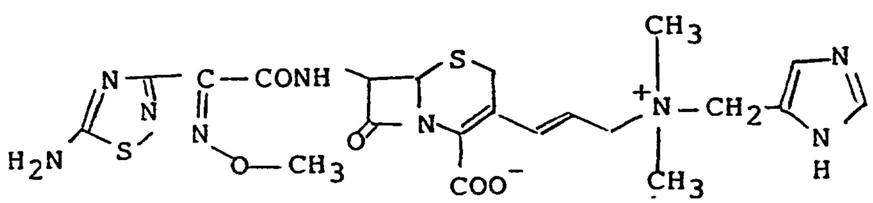

500 Mg of the compound obtained in Experiment 5 were suspended in 10 mℓ of ethyl ether, and then 125 mg of 4-(dimethylaminomethyl)imidazole were added thereto, followed by stirring for one hour. To this reaction solution were added 50 mℓ of ethyl ether. The resulting precipitate was recovered by filtration to yield 382 mg of a yellow powder.

This yellow powder was added to a liquid mixture of 2.5 mℓ of trifluoroacetic acid and 2.5 mℓ of anisole, followed by stirring under ice-cooling for 3 hours. To this reaction solution were added 50 mℓ of isopropyl ether, and the resulting precipitate was recovered by filtration. This precipitate was suspended in 2 mℓ of water. The pH of the suspension was adjusted to 7 - 8 by an addition of sodium hydrogencarbonate. The insoluble matters were removed by filtration. The filtrate was purified by a reversed-phase silica gel chromatography to yield 23 mg of the objective compound.

Compounds in the following Examples 13 - 16 were prepared in the same manner as described in Examples 10 - 12.

Example 13

7β-[2-(5-Amino-1,2,4-thiadiazol-3-yl)-(Z)-2methoxyiminoacetamido]-3-[(E)-3[(thiazol-4-yl)methyldimethyl-ammonio]-1-propen-1-yl]-3-cephem-4-carboxylate

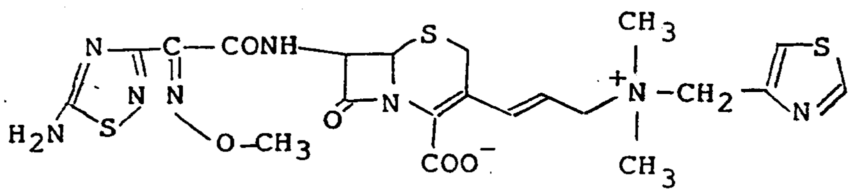

500 Mg of the compound obtained in the Experiment 5 were reacted with 126 mg of 4-dimethylaminomethyl thiazol, followed by removing the protective group to yield 33 mg of the objective compound.

Example 14

7β-[2-(5-Amino-1,2,4-thiadiazol-3-yl)-(Z)-2-methoxyiminoacetamido]-3-[(E)-3-[(pyrazol-3-yl)methyldimethyl-ammonio]-1-propen-1-yl]-3-cephem-4-carboxylate

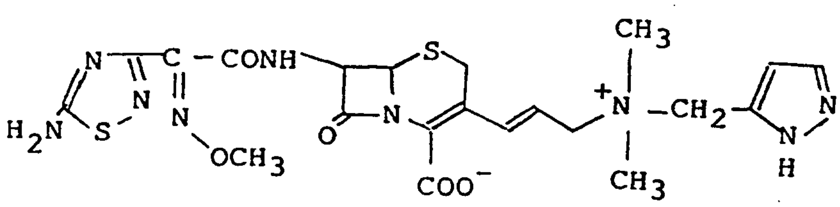

500 Mg of the compound obtained in the Experiment 5 were reacted with 120 mg of 3-dimethylaminomethyl pyrazole, followed by removing the protective group to yield 200 mg of the objective compound.

Example 15

7β-[2-(5-Amino-1,2,4-thiadiazol-3-yl)-(Z)-2methoxyiminoacetamido]-3-[(E)-3-[(2-aminothiazol-4-yl)-methyldi-methylammonio]-1-propen-1-yl]-3-cephem-4-carboxylate

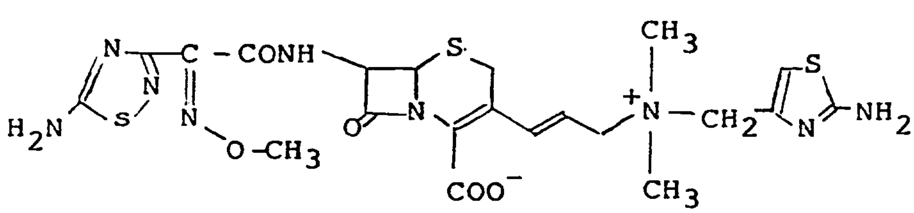

500 Mg of the compound obtained in the Experiment 5 were reacted with 140 mg of 2-amino-4-dimethylaminomethylthiazole, followed by removing the protective group to yield 58 mg of the objective compound.

Example 16

7β-[2-(5-Amino-1,2,4-thiadiazol-3-yl)-(Z)-2-methoxyiminoacetamido]-3-[(E)-3-[(furan-3-yl)-methyldimethylammonio]-1-propen-1-yl]-3-cephem-4-carboxylate

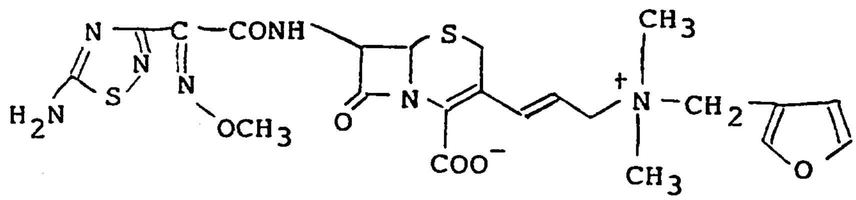

500 Mg of the compound obtained in the Experiment 5 were reacted with 125 mg of 3-dimethylaminomethyl furan, followed by removing the protective group to yield 35 mg of the objective compound.

Example 17

7β-[2-(5-Amino-1,2,4-thiadiazol-3-yl)-(Z)-2-methoxyiminoacetamido]-3-[(E)-3-[N,N-dimethyl-N-(2-carbamoylethyl)ammonio]-1-propen-1-yl]-3-cephem-4-carboxylate

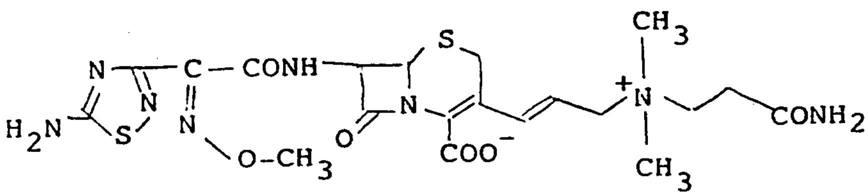

500 Mg of the compound obtained in the Experiment 5 were reacted with 127 mg of 3-dimethylaminopropionamide, and the protective group was removed to yield 12 mg of the objective compound.

Example 18

7β-[2-(5-Amino-1,2,4-thiadiazol-3-yl)-(Z)-2-methoxyiminoacetamido]-3-[(E)-3-(N,N-dimethyl-N-hydrazinocarbonylmethylammonio)-1-propen-1-yl]-3-cephem-4-carboxylate

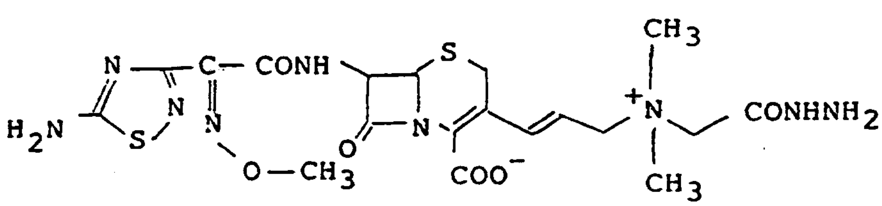

500 Mg of the compound obtained in the Experiment 5 were reacted with 110 mg of N,N-dimethylacetohydrazine, and the protective group was removed to yield 14 mg of the objective compound.

TABLE 1 (List of physical value)

| Experiment No. | Infra-red Absorption Spectrum ($cm^{-1}$, Nujol) | NMR (δ) |
|---|---|---|
| 1 | 1765, 1715, 1680, 1600, 1575 | (DMSO-$d_6$) 3.5 -3.7 (2H, br), 3.74 (3H, s), 3.98 (3H, s), 4.46 (2H, s), 5.15 (1H, d, J=5Hz), 5.19 (2H, s), 5.89 (1H, dd, J=5 Hz, 8Hz) 6.90 (2H, d, J=9Hz), 7.29 (15H, s), 7.34 (2H, d, J=9 Hz), 9.52 (1H, d, J=8Hz), 9.97 (2H, s) |
| 2 | 1765, 1715, 1670, 1600, 1580 | ($CDCl_3$) 3.42 (1H, d, J=18Hz), 3.70 (1H, d, J=18Hz), 3.76 (3H, s), 4.08 (3H, s), 4.23 (1H, d, J=9Hz), 4.38 (1H, d, J=9Hz), 4.94 (1H, d, J=5Hz), 5.16 (2H, s), 5.82 (1H, dd, J=5Hz, 9Hz), 6.79 (1H, d, J=9Hz), 6.80 (2H, d, J=9Hz), 7.22 (15H, m), 7.27 (2H, d, J=9Hz), 7.55 (1H, s) |
| 3 | 1760, 1700, 1670, 1600, 1575 | ($CDCl_3$) 3.2-3.3 (1H, br), 3.79 (3H, s), 4.0 (3H, br), 4.7 - 4.8 (1H, br), 4.8-4.9 (1H, br), 5.02 (1H, d, J=4.8 Hz), 5.05-5.2 (1H, br), 5.7-5.85 (1H, br), 5.83 (1H, dd, J=4.8 Hz, 8.8 Hz), 6.80 (2H, d, J=8.4 Hz), 7.0-7.1 (2H, b), 7.2-7.35 (15H, m), 7.65-7.85 (15H, m) |
| 4 | 1760, 1715, 1655, 1590 | ($CDCl_3$) 3.32 (1H, d, J=17Hz), 3.52 (1H, d, J=17Hz), 3.81 (3H, s), 4.11 (2H, d, J=8.1 Hz), 4.14 (3H, s), 5.08 (1H, d, J=4.9 Hz), 5.16 (2H, s), 5.74 (1H, dt, J=11.4Hz, 8.1 Hz), 5.96 (1H, dd, J=4.9 Hz, 9.2 Hz), 6.25 (1H, d, J=11.4Hz), 6.73 (1H, d, J=9.2 Hz), 6.88 (2H, d, J=8.8 Hz), 7.25 (2H, d, J=8.8 Hz), 7.2-7.4 (15H, m), 7.53 (1H, s) |

| Experiment No. | Infra-red Absorption Spectrum ($cm^{-1}$, Nujol) | N M R (δ) |
|---|---|---|
| 5 | 1760, 1715, 1670, 1600, 1530 | ($CDCl_3$) 3.51 (1H, d, J=17.6Hz), 3.61 (1H, d, J=17.6Hz), 3.81 (3H, s), 3.93 (2H, d, J=8.4 Hz), 4.18 (3H, s), 5.03 (1H, d, J=4.8 Hz), 5.19(1H, d, J=11.7Hz), 5.25 (1H, d, J=11.7Hz), 5.92 (1H, dd, J=4.8 Hz, 8.8 Hz), 6.12 (1H, dt J=8.4 Hz, 15.8Hz), 6.79 (1H, d, J=8.8 Hz), 6.90 (2H, d, J=8.8 Hz), 7.00 (1H, d, J=15.8Hz), 7.15-7.3 (17H, m) |
| 6 | ——— | ($DMSO-d_6$) 1.12 (3H, t, J=8Hz), 3.72 (3H, s), 4.12 (2H, q, J=8Hz), 4.43 (2H, bs), 5.04-5.25 (3H, m), 5.77 (1H, dd, J=5Hz, 10 Hz), 6.85 (2H, d, J=8Hz), 7.1-7.4(17H, m), 8.44 (1H, d, J=10 Hz) |
| 7 | 1610, 1690, 1710, 1760 | ($DMSO-d_6$) 1.22 (3H, t, J=8Hz), 3.75 (3H, s), 3.96-4.30 (5H,m), 5.11 (2H, s), 5.22 (1H, d, J=5Hz), 5.82 (1H, dd, J=5Hz, 10Hz), 6.28 (1H, d, J=13Hz), 6.92 (2H, d, J=8Hz), 7.2-7.5 (17H, m), 9.52 (1H, d, J=10Hz) |

| Example No. | Infra-red Absorption Spectrum (cm-1. Nujol) | N M R (δ) |
|---|---|---|
| 1 | 1755, 1650, 1615, 1580 | ($DMSO-d_6$) 3.14 (3H, s), 3.15 (3H, s), 3.47 (1H, d, J=17Hz), 3.65 (1H, d, J=17Hz), 3.91 (3H, s), 4.02 (2H, s), 4.16 (2H, d, J=7.7 Hz), 5.04 (1H, d, J=4.8 Hz), 5.70 (1H, dt, J=7.7 Hz, 15.8Hz), 5.63 (1H, dd, J=4.8 Hz, 8.4 Hz), 7.16 (1H, d, J=15.8Hz), 7.64 (1H, s), 8.14 (2H, s), 8.23 (1H, s), 9.51 (1H, d, J=8.4 Hz) |

| Example No. | Infra-red Absorption Spectrum (cm-1, Nujol) | NMR (δ) |
|---|---|---|
| 2 | 1755, 1650, 1595 | ($DMSO-d_6$) 3.05 (3H, s), 3.06 (3H, s), 3.45 (1H, d, J=17.2Hz), 3.51 (2H, s), 3.64 (1H, d, J=17.2Hz), 3.91 (3H, s), 4.15 (2H, m), 5.03 (1H, d, J=4.8 Hz), 5.65 (1H, m), 5.62 (1H, dd, J=4.8 Hz, 8.8 Hz), 7.13 (1H, d, J=15.8Hz), 8.14 (2H, s), 9.51 (1H, d, J=8.8 Hz) |
| 3 | 1760, 1685, 1670, 1625, 1590 | ($DMSO-d_6$) 1.24 (6H, t, J=7.0 Hz), 3.4-3.5 (5H, m), 3.71 (1H, d, J=16.1Hz), 3.90 (2H, s), 3.91 (3H, s), 4.1 (2H, m), 5.06 (1H, d, J=4.0 Hz), 5.65 (1H, m), 5.7 (1H, m), 7.14 (1H, d, J=15.0Hz), 7.70 (1H, s), 8.04 (1H, s), 8.12 (2H, s), 9.51 (1H, d, J=8.8 Hz) |

| Example No. | Infra-red Absorption Spectrum (cm$^{-1}$, Nujol) | N M R (δ) |
|---|---|---|
| 4 | 1760, 1665, 1625, 1595 | (DMSO-$d_6$) 2.20 (6H, s), 2.65 (2H, t, J=5.9 Hz), 3.00 (6H, s), 3.33 (2H, t, J=5.9 Hz), 3.46 (1H, d, J=17.2Hz), 3.66 (1H, d, J=17.2Hz), 3.91 (3H, s), 4.01 (2H, d, J=7.0 Hz), 5.04 (1H, d, J=5.1 Hz), 5.63 (1H, dd, J=5.1 Hz, 8.4 Hz), 5.69 (1H, dt, J=7.0 Hz, 15.4Hz), 7.17(1H, d, J=15.4Hz),8.12 (2H, s), 9.50 (1H, d, J=8.4 Hz) |
| 5 | ——— | ($D_2O$) 3.24 (6H, s), 4.21 (2H, m), 4.21 (3H, s), 5.40 (1H, d, J=5Hz), 5.97 (1H, d, J=5Hz), 6.18 (1H, m), 7.13 (1H, d, J=14.8Hz) |
| 6 | 1760, 1690, 1615, 1595 | ($D_2O$) 1.45 (3H, t, J=7.5 Hz), 3.34 (3H, s), 3.35 (3H, s), 3.76 (1H, d, J=17Hz), 3.82 (1H, d, J=17Hz), 4.14 (2H, s), 4.33 (1H, d, J=7.7 Hz), 4.46 (2H, q, J=7.5 Hz), 5.38 (1H, d, J=4.8 Hz), 5.96 (1H, d, J=4.8 Hz), 6.05 (1H, dt, J=7.7 Hz, 15.75 Hz), 7.03 (1H, d, J=15.75 Hz) |

| Example No. | Infra-red Absorption Spectrum ($cm^{-1}$, Nujol) | NMR (δ) |
|---|---|---|
| 7 | 1760, 1720, 1660, 1620, 1595 | ($D_2O$) 2.32 (3H, s), 3.30 (8H, s), 3.75 (1H, d, J=17Hz), 3.81 (1H, d, J=17Hz), 4.19 (3H, s), 4.30 (2H, d, J=7.7 Hz), 5.37 (1H, d, J=4.8 Hz), 5.95 (1H, d, J=4.8 Hz), 6.00 (1H, dd, J=7.7 Hz, 15.7Hz), 6.96 (1H, d, J=15.7Hz) |
| 8-A | 1764, 1665, 1600, 1532 | ($D_2O$) 3.17 (2H, m), 3.17 (6H, s), 3.30 (2H, m), 3.75 (2H, br), 4.10 (2H, m), 4.14 (3H, s), 5.33 (1H, d, J=4Hz), 5.90 (1H, d, J=4Hz), 6.11 (1H, m), 6.97 (1H, d, J=15Hz) |
| 8-B | 1764, 1665, 1600, 1532 | ($D_2O$) 3.17 (2H, br), 3.17 (6H, s), 3.44 (2H, br), 3.44 (2H, br), 4.04 (2H, m), 4.15 (3H, s), 5.33 (1H, d, J=4Hz), 5.92 (1H, d, J=4Hz), 5.94 (1H, m), 6.61 (1H, d, J=12Hz) |
| 9 | 1760, 1685, 1655, 1590 | (DMSO-$d_6$) 3.17 (3H, s), 3.20 (3H, s), 3.27 (1H, d, J=16.9Hz), 3.53 (1H, d, J=16.9Hz), 3.91 (3H, s), 3.97 (2H, s), 4.05 (1H, dd, J=13.9Hz, 8.1 Hz), 4.20 (1H, dd, J=13.9Hz, 7.1 Hz), 5.06 (1H, d, J=4.8 Hz), 5.55-5.70 (1H, m), 5.67 (1H, dd, J=8.4 Hz, 4.8 Hz), 6.72 (1H, d, J=11.4Hz), 7.61 (1H, s), 8.12 (1H, s), 8.12 (2H, s), 9.52 (1H, d, J=8.4 Hz) |
| 10 | 1760, 1660, 1600 | ($D_2O$) 3.33 (6H, s), 3.78 (1H, d, J=17.2Hz), 3.85 (1H, d, J=17.2Hz), 4.19 (3H, s), 4.25~4.33 (2H, m), 5.06 (2H, s), 5.39 (1H, d, J=4.8 Hz), 5.96 (1H, d, J=4.8 Hz), 6.12 (1H, dd, J=15.8Hz, 7.2Hz), 7.10 (1H, d, J=15.8Hz) 9.25 (1H, s) |

| Example No. | Infra-red Absorption Spectrum ($cm^{-1}$, Nujol) | N M R (δ) |
|---|---|---|
| 11 | ———— | ($DMSO-d_6$) 3.03 (6H, s), 3.92 (3H, s), 4.1 ~4.3 (2H, m), 4.85 (2H, s), 5.03 (1H, d, J=5Hz), 5.62 (1H, dd, J=5Hz, 10Hz), 5.7 ~ 5.9 (1H, m), 7.24 (1H, d, J=17Hz), 8.18 (2H, s), 9.51 (1H, d, J=10Hz), 9.90 (1H, s) |
| 12 | 1770, 1670, 1610 | ($D_2O$) 3.08 (6H, s), 3.77 (1H, d, J=17.2Hz), 3.85 (1H, d, J=17.2Hz), 4.06 (2H, d, J=7.7 Hz), 4.18 (3H, s), 4.50 (2H, s), 5.37 (1H, d, J=4.8 Hz), 5.95 (1H, d, J=4.8 Hz), 6.09 (1H, dt, J=15.8 Hz, 7.7 Hz), 7.00 (1H, d, J=15.8Hz), 7.57 (1H, s), 7.93 (1H, s) |
| 13 | 1770, 1660, 1600 | ($D_2O$) 3.14 (6H, s), 3.77 (1H, d, J=17.2Hz), 3.85 (1H, d, J=17.2Hz), 4.14 (2H, d, J=7.2 Hz), 4.18 (3H, s), 5.37 (1H, d, J=4.7 Hz), 5.95 (1H, d, J=4.7 Hz), 6.12 (1H, dt, J=15.3Hz, 7.2 Hz), 7.03 (1H, d, J=15.3Hz), 8.09 (1H, d, J=1.9 Hz), 9.21 (1H, d, J=1.9 Hz) |
| 14 | 1765, 1660, 1600 | ($D_2O$) 3.10 (6H, s), 3.77 (1H, d, J=17.2Hz), 3.84 (1H, d, J=17.2Hz), 4.10 (2H, d, J=7.0 Hz), 4.17 (3H, s), 4.58 (2H, s), 5.37 (1H, d, J=4.8 Hz), 5.94 (1H, d, J=4.8 Hz), 6.09 (1H, dt, J=15.4 Hz, 7.0 Hz), 6.72 (1H, d, J=2.6 Hz), 7.02 (1H, d, J=15.4Hz), 7.91 (1H, d, J=2.6 Hz) |

| Example No. | Infra-red Absorption Spectrum ($cm^{-1}$, Nujol) | NMR (δ) |
|---|---|---|
| 15 | 1770, 1660, 1600 | ($D_2O$) 3.12 (3H, s), 3.77 (1H, d, J=17.2Hz), 3.83 (1H, d, J=17.2Hz), 4.11 (2H, d, J=7.3 Hz), 4.18 (3H, s), 4.37 (2H, s), 5.37 (1H, d, J=5.0 Hz), 5.95 (1H, d, J=5.0 Hz), 6.07 (1H, dt, J=15.4 Hz, 7.3 Hz), 7.02 (1H, d, J=15.4Hz), 7.10 (1H, s) |
| 16 | 1770, 1670, 1600 | ($D_2O$) 3.08 (6H, s), 3.77 (1H, d, J=17.4Hz), 3.84 (1H, d, J=17.4Hz), 4.07 (1H, d, J=7.3 Hz), 4.18 (3H, s), 4.45 (2H, s), 5.37 (1H, d, J=4.9 Hz), 6.08 (1H, dt, J=15.4Hz, 7.3 Hz), 6.73 (1H, d, J=1.1 Hz), 7.00 (1H, d, J=15.4Hz), 7.74 (1H, d, J=1.1 Hz), 7.91 (1H, s) |

| Example No. | Infra-red Absorption Spectrum ($cm^{-1}$, Nujol) | NMR ($\delta$) |
|---|---|---|
| 17 | — | ($D_2O$) 2.76~3.24 (4H, m), 3.12 (6H, s), 3.28~3.82 (4H, m), 4.14 (3H, s), 5.32 (1H, d, J=5Hz), 5.90 (1H, d, J=5Hz), 6.07 (1H, m), 6.98 (1H, d, J=15.5Hz) |
| 18 | — | ($D_2O$) 3.24 (6H, s), 3.67 (1H, d, J=17Hz), 3.73 (1H, d, J=17Hz), 3.97 (2H, s), 4.10 (3H, s), 4.21 (2H, d, J=7.7 Hz), 5.34 (1H, d, J=4.1 Hz), 5.88 (1H, d, J=4.1 Hz), 5.97 (1H, dt, J=15.4 Hz, 7.7 Hz), 6.95 (1H, d, J=16.4Hz) |

Effect of the Invention

TABLE 2 (Antibacterial activities)

| Test compound \ Test bacteria | MIC (μg/mℓ) | | | | | |
|---|---|---|---|---|---|---|
| | Staph. aureus 209-P | Escher. coli NIHJ | Kleb Pneumoniae EK-6 | Ser. marcescens ES-75 | Morg. morganii EP-14 | Pseud. aeruginosa EP-01 |
| Example 1 | 0.4 | 0.05 | 0.05 | 0.1 | 0.05 | 0.8 |
| 3 | 0.2 | 0.05 | ≦0.025 | 0.1 | 0.05 | 1.56 |
| 4 | 0.4 | 0.05 | 0.05 | 0.1 | 0.1 | 1.56 |
| 6 | 0.2 | 0.1 | 0.05 | 0.2 | 0.05 | 1.56 |
| 7 | 0.4 | 0.05 | 0.05 | 0.1 | 0.1 | 1.56 |
| 8 -A | 0.2 | ≦0.025 | ≦0.025 | 0.1 | 0.05 | 0.8 |
| 10 | 0.40 | ≦0.025 | ≦0.025 | 0.10 | 0.05 | 0.80 |
| 11 | 0.20 | ≦0.025 | ≦0.025 | 0.05 | ≦0.025 | 1.56 |
| 12 | 0.20 | 0.05 | 0.05 | 0.10 | 0.05 | 1.56 |
| 13 | 0.20 | ≦0.025 | ≦0.025 | 0.05 | ≦0.025 | 3.13 |
| 14 | 0.20 | ≦0.025 | ≦0.025 | 0.10 | 0.05 | 3.13 |

| Test bacteria / Test compound | MIC (μg/mℓ) | | | | | |
|---|---|---|---|---|---|---|
| | Staph. aureus 209-P | Escher. coli NIHJ | Kleb Pneumoniae EK-6 | Ser. marcescens EC-75 | Morg. morganii EP-14 | Pseud. aeruginosa EP-01 |
| Example 15 | 0.20 | ≦0.025 | 0.03 | 0.03 | 0.05 | 3.13 |
| 16 | 0.20 | ≦0.025 | ≦0.025 | 0.05 | ≦0.025 | 3.13 |
| 17 | 0.2 | ≦0.025 | 0.1 | 0.05 | 0.05 | 0.8 |

## Claims

**Claims for the following Contracting States : BE, CH, DE, FR, GB, IT, LI, LU, NL, SE**

1. A cephalosporin derivative represented by the formula:

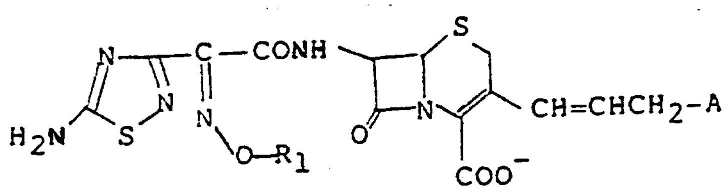

(I)

wherein $R_1$ represents a $C_1$-$C_4$ alkyl group, and A is a group of the following formula:

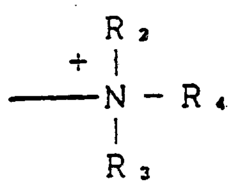

wherein $R_2$ and $R_3$ are the same or different $C_1$-$C_4$ alkyl group, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent; or its pharmacologically acceptable salt.

2. A cephalosporin derivative or its pharmacologically acceptable salt according to claim 1, comprising a compound or its pharmacologically acceptable salt wherein A in the formula (I) is a group represented by one of the following formulae:

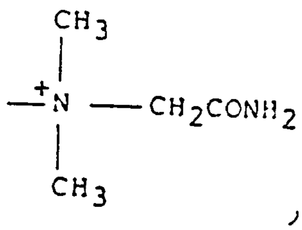,
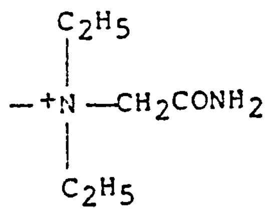,
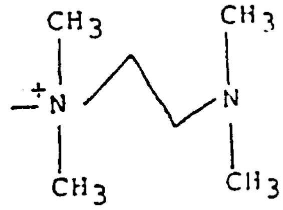,

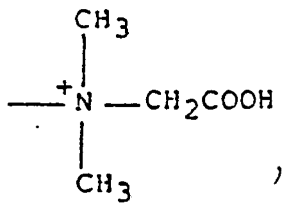,
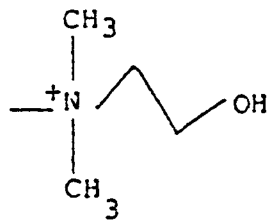,
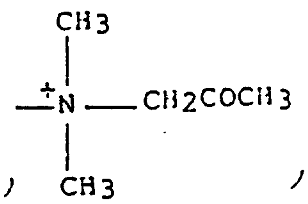,

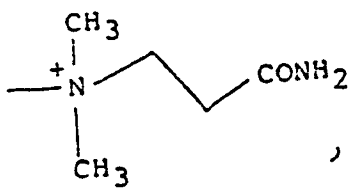,
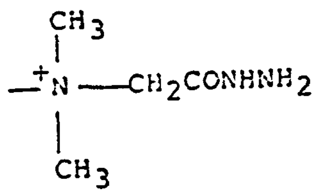,
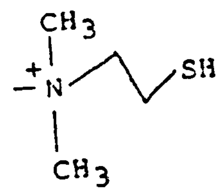,

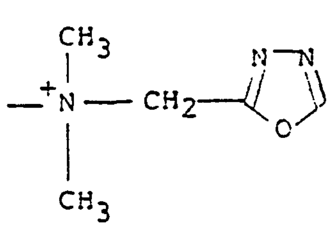,
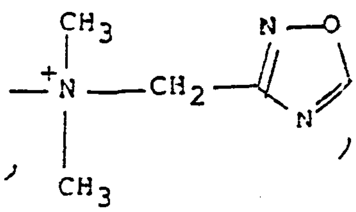,

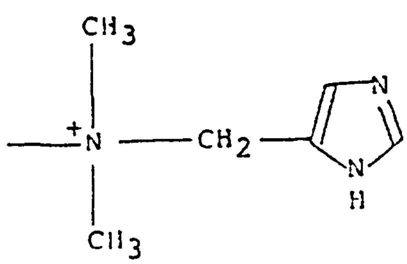,
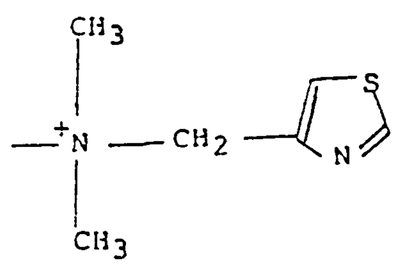,

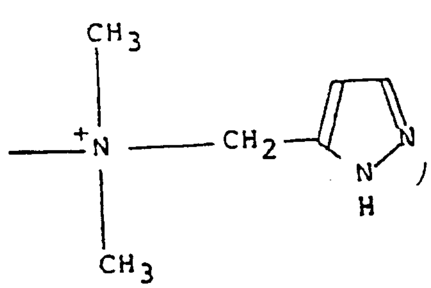,
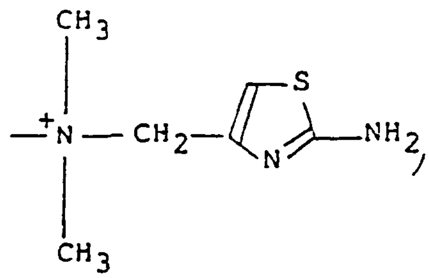,

**3.** A cephalosporin derivative or its pharmacologically acceptable salt according to claim 1, wherein said cephalosporin derivative or its pharmacologically acceptable salt is 7β-[2-(5-amino-1,2,4-thiadiazol-3-yl)-(Z)-2-methoxyimino-acetamido]-3-[(E)-3-(carbamoylmethyldimethylammonio)-1-propene-1-yl]-3-cephem-4-carboxylate or its pharmacologically acceptable salt.

**4.** A cephalosporin derivative or its pharmacologically acceptable salt according to claim 1, wherein said cephalosporin derivative or its pharmacologically acceptable salt is 7β-[2-(5-amino-1,2,4-thiadiazol-3-yl)-(Z)-2-methoxyimino-acetamido]-3-[(E)-3-(2-hydroxyethyldimethylammonio)-1-propene-1-yl]-3-cephem-4-carboxylate or its pharmacologically acceptable salt.

**5.** A cephalosporin derivative or its pharmacologically acceptable salt according to claim 1, therein said cephalosporin derivative or its pharmacologically acceptable salt is 7β-[2-(5-amino-1,2,4-thiadiazol-3-yl)-(Z)-2-methoxyimino-acetamido]-3-[(E)-3-[(1,3,4-oxadiazol-2-yl) methyldimethylammonio]-1-propene-1-yl]-3-cephem-4-carboxylate or its pharmacologically acceptable salt.

**6.** A cephalosporin derivative or its pharmacologically acceptable salt according to claim 1, wherein said cephalosporin derivative or its pharmacologically acceptable salt is 7β-[2-(5-amino-1,2,4-thiadiazol-3-yl)-(Z)-2-methoxyiminoacetamido]-3-[(E)-3-(2-oxopropyldimethylammonio)-1-propene-1-yl]-3-cephem-4-carboxylate or its pharmacologically acceptable salt.

**7.** A process for preparing a cephalosporin derivative represented by the formula:

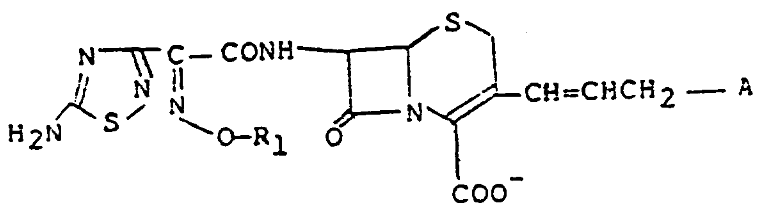

(I)

wherein $R_1$ is a $C_1$-$C_4$ alkyl group, and A represents a quaternary ammonium salt corresponding to A', or its pharmacologically acceptable salt, which comprises reacting a compound represented by the formula:

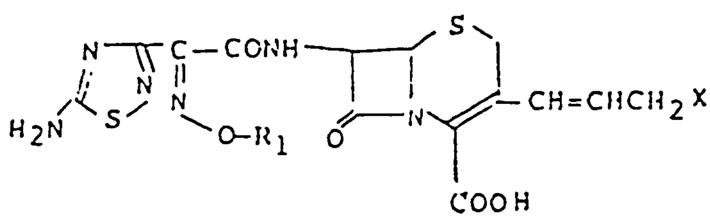

(II)

wherein $R_1$ is as defined above, and X is halogen atom, a compound with the amino and/or carboxyl group(s) of the compound being protected by a protective group, or a salt of one of these compounds, with a compound or its salt represented by the formula:

A' (III)

wherein A' is
a group of the following formula:

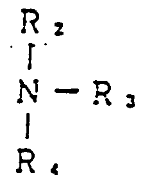

where $R_2$ and $R_3$ are the same or different $C_1$-$C_4$ alkyl group, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino, carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent;
and optionally removing the protective group.

**8.** An anti-bacterial agent comprising:
an effective amount of a cephalosporin derivative represented by the formula:

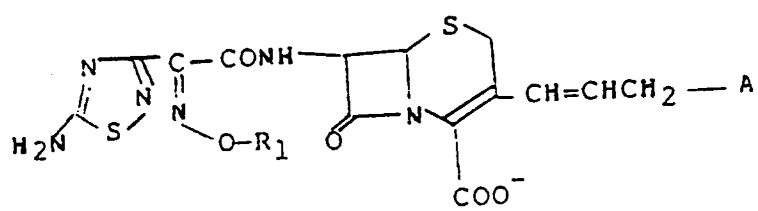

(I)

wherein $R_1$ represents a $C_1$-$C_4$ alkyl group, and A is
a group of the following formula:

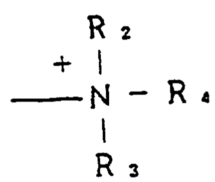

where $R_2$ and $R_3$ are the same or different $C_1$-$C_4$ alkyl group, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino, carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent; or its pharmacologically acceptable salt; and a pharmacologically acceptable excipient.

**Claims for the following Contracting State : AT**

**1.** A process for preparing a cephalosporin derivative represented by the formula:

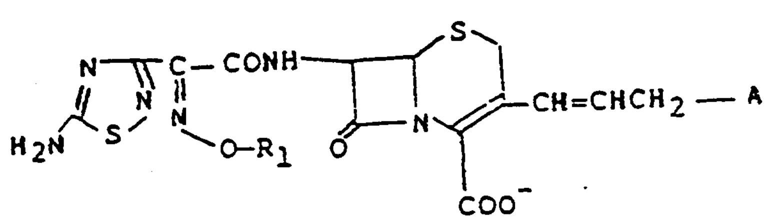

(I)

wherein $R_1$ is a $C_1$-$C_4$ alkyl group, and A represents a quaternary ammonium salt corresponding to A', or its pharmacologically acceptable salt, which comprises reacting a compound represented by the formula:

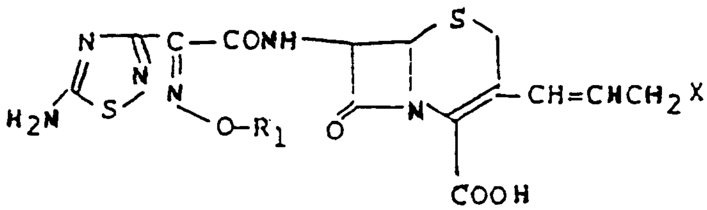

(II)

wherein $R_1$ is as defined above, and X is halogen atom, a compound with the amino and/or carboxyl group(s) of the compound being protected by a protective group, or a salt of one of these compounds, with a compound or its salt represented by the formula:

A' (III)

wherein A' is

a group of the following formula:

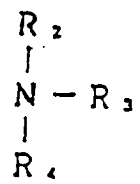

where $R_2$ and $R_3$ are the same or different $C_1$-$C_4$ alkyl group, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino, carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent;

and optionally removing the protective group.

**2.** A process as claimed in claim 1, wherein A in the formula (I) is a group represented by one of the following formulae:

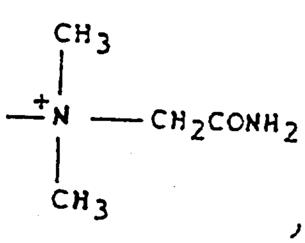 , 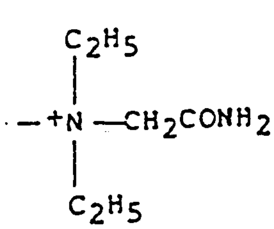 , 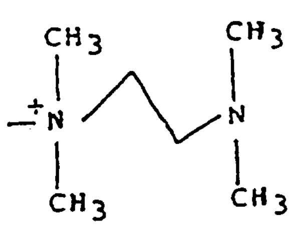 ,

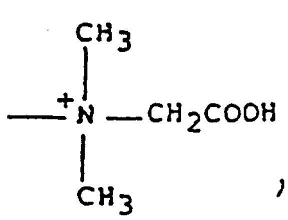 , 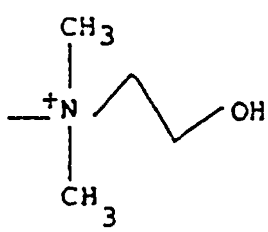 , 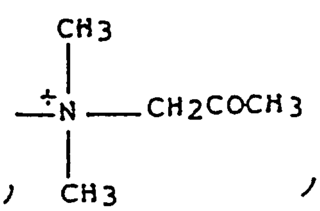 ,

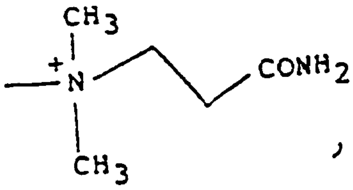
,
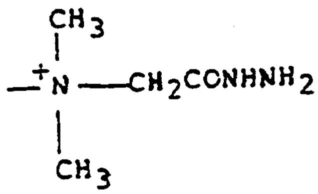
,
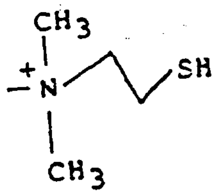
;

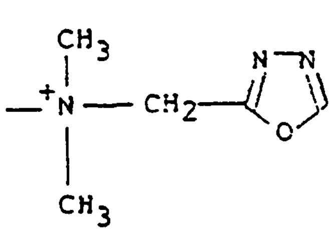
,
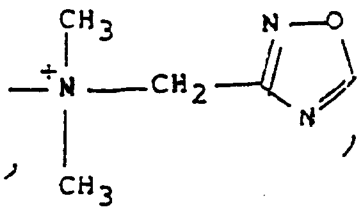
,

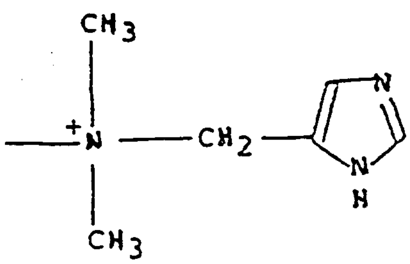
,
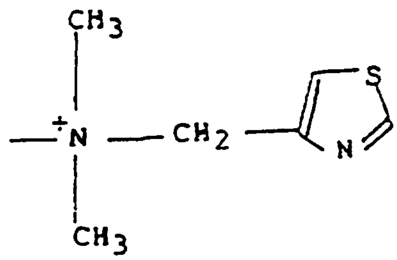
,

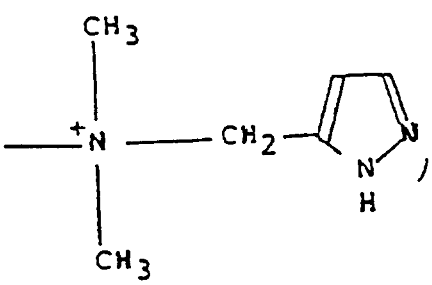
,
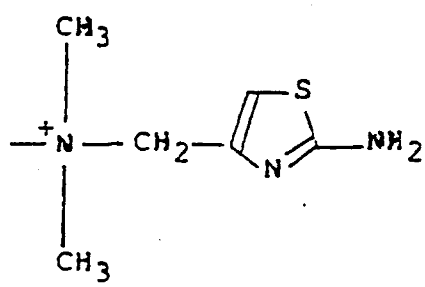
.

3. A process as claimed in claim 1, wherein said cephalosporin derivative or its pharmacologically acceptable salt is 7β-[2-(5-amino-1,2,4-thiadiazol-3-yl)-(Z)-2-methoxyimino-acetamido]-3-[(E)-3-(carbamoylmethyldimethylammonio)-1-propene-1-yl]-3-cephem-4-carboxylate or its pharmacologically acceptable salt.

4. A process as claimed in claim 1, wherein said cephalosporin derivative or its pharmacologically acceptable salt is 7β-[2-(5-amino-1,2,4-thiadiazol-3-yl)-(Z)-2-methoxyimino-acetamido]-3-[(E)-3-(2-hydroxyethyldimethylammonio)-1-propene-1-yl]-3-cephem-4-carboxylate or its pharmacologically acceptable salt.

5. A process as claimed in claim 1, wherein said cephalosporin derivative or its pharmacologically acceptable salt is 7β-[2-(5-amino-1,2,4-thiadiazol-3-yl)-(Z)-2-methoxyimino-acetamido]-3-[(E)-3-[(1,3,4-oxadiazol-2-yl)-methyldimethylammonio]-1-propene-1-yl]-3-cephem-4-carboxylate or its pharmacologically acceptable salt.

6. A process as claimed in claim 1, wherein said cephalosporin derivative or its pharmacologically acceptable salt is 7β-[2-(5-amino-1,2,4-thiadiazol-3-yl)-(Z)-2-methoxyiminoacetamido]-3-[(E)-3-(2-oxopropyldimethylammonio)-1-propene-1-yl]-3-cephem-4-carboxylate or its pharmacologically acceptable salt.

7. The use of a cephalosporin derivative represented by the formula:

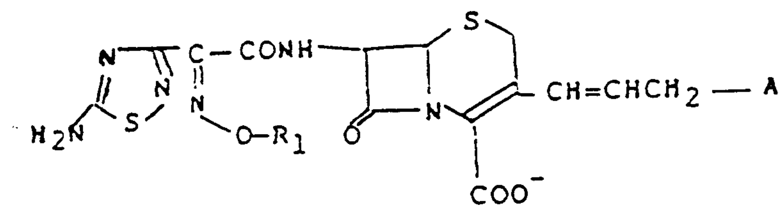

(I)

wherein $R_1$ represents a $C_1$-$C_4$ alkyl group, and A is a group of the following formula:

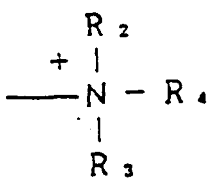

where $R_2$ and $R_3$ are the same or different $C_1$-$C_4$ alkyl group, $R_4$ is a $C_1$-$C_4$ alkyl group substituted with a group selected from the group consisting of a carbamoyl, carboxyl, $C_1$-$C_4$ alkyl-substituted amino, mercapto, $C_1$-$C_4$ alkanoyl, hydroxyl, or hydrazinocarbonyl group; a $C_1$-$C_4$ alkyl group substituted with a 5-membered heterocyclic radical which may have: amino, carboxyl, $C_1$-$C_4$ alkyl, carboxy $C_1$-$C_4$ alkyl, carbamoyl, hydroxyl, $C_1$-$C_4$ alkyl-substituted amino and hydroxy $C_1$-$C_4$ alkyl group as a substituent; or its pharmacologically acceptable salt;
for manufacturing an anti-bacterial agent.

## Patentansprüche
## Patentansprüche für folgende Vertragsstaaten : BE, CH, DE, FR, GB, IT, LI, LU, NL, SE

1. Cephalosporin-Derivat, dargestellt durch die Formel:

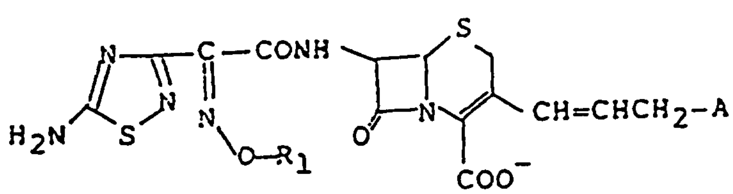

(I)

worin $R_1$ eine $C_{1-4}$-Alkyl-Gruppe darstellt und A ist: eine Gruppe der folgenden Formel

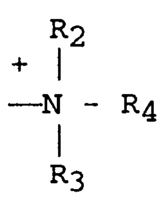

worin $R_2$ und $R_3$ gleiche oder verschiedene $C_{1-4}$-Alkyl-Gruppen sind, $R_4$ eine $C_{1-4}$-Alkyl-Gruppe ist, die substituiert ist mit einer Gruppe, ausgewählt aus der Gruppe bestehend aus einer Carbamoyl-, Carboxyl-, $C_{1-4}$-Alkyl-substituierten Amino-, Mercapto-, $C_{1-4}$-Alkanoyl-, Hydroxyl- oder Hydrazinocarbonyl-Gruppe; oder eine $C_{1-4}$-Alkyl-Gruppe ist, die mit einer 5-gliedrigen heterocyclischen Gruppe substituiert ist, welche als Substituenten haben kann: Amino, Carboxyl, $C_{1-4}$-Alkyl, Carboxy-$C_{1-4}$-alkyl, Carbamoyl, Hydroxyl, $C_{1-4}$-Alkyl-substituiertes Amino und Hydroxy-$C_{1-4}$-alkyl; oder dessen pharmakologisch annehmbare Salze.

**2.** Cephalosporin-Derivat oder dessen pharmakologisch annehmbare Salze gemäß Anspruch 1, umfassend eine Verbindung oder deren pharmakologisch annehmbares Salz, in der A in der Formel (I) eine Gruppe ist, die durch eine der folgenden Formeln dargestellt wird:

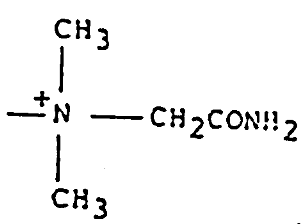 , 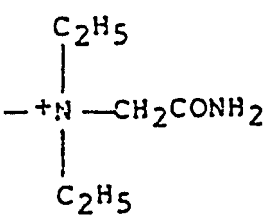 , 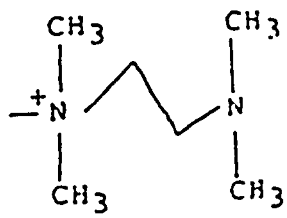 ,

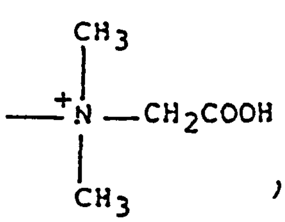 , 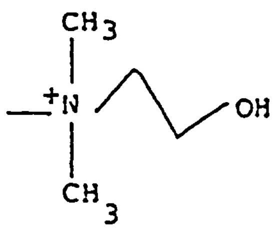 , 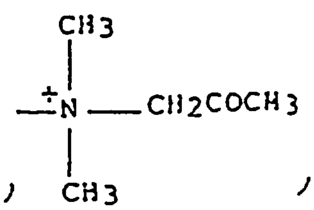 ,

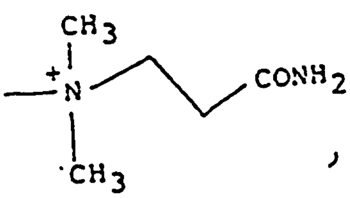 , 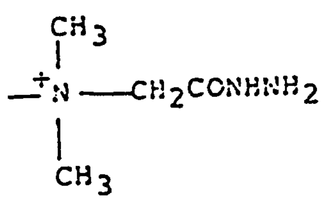 , 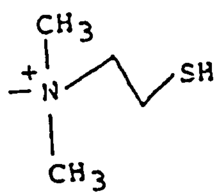 ,

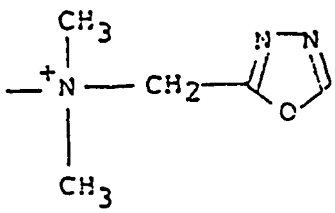 , 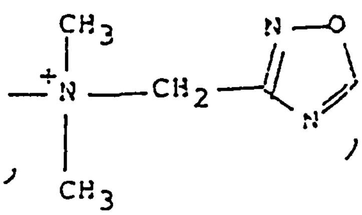 ,

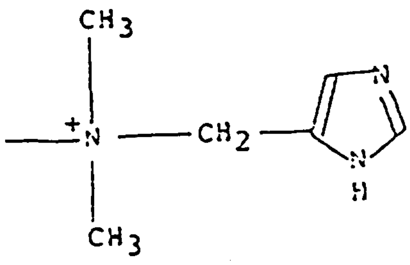 , 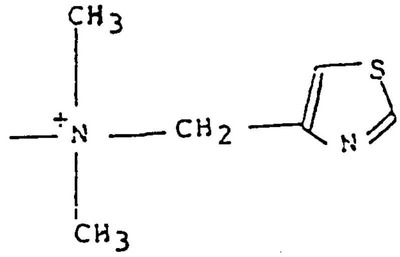 ,

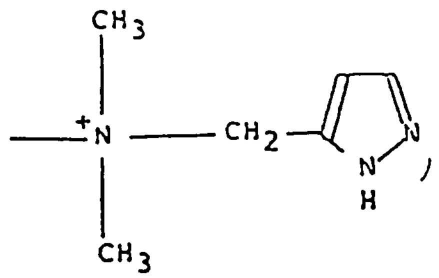

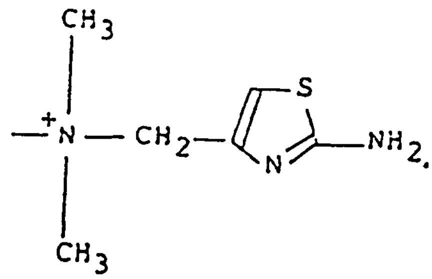

3. Cephalosporin-Derivat oder dessen pharmakologisch annehmbare Salze gemäß Anspruch 1, dadurch **gekennzeichnet,** daß das Cephalosporin-Derivat oder dessen pharmakologisch annehmbares Salz 7β-[2-(5-Amino-1,2,4-thiadiazol-3-yl)-(Z)-2-methoxyiminoacetamido]-3-[(E)-3-(carbamoylmethyldimethylammonium)-1-propen-1-yl]-3-cephem-4-carboxylat oder ein pharmakologisch annehmbares Salz davon ist.

4. Cephalosporin-Derivat oder dessen pharmakologisch annehmbares Salz gemäß Anspruch 1, dadurch **gekennzeichnet,** daß dieses Cephalosporin-Derivat oder dessen pharmakologisch annehmbares Salz 7β-[2-(5-Amino-1,2,4-thiadiazol-3-yl)-(Z)-2-methoxyiminoacetamido]-3-[(E)-3-(2-hydroxyethyldimethylammonium)-1-propen-1-yl]-3-cephem-4-carboxylat oder ein pharmakologisch annehmbares Salz davon ist.

5. Cephalosporin-Derivat oder dessen pharmakologisch annehmbares Salz gemäß Anspruch 1, dadurch **gekennzeichnet,** daß dieses Cephalosporin-Derivat oder dessen pharmakologisch annehmbares Salz 7β-[2-(5-Amino-1,2,4-thiadiazol-3-yl)-(Z)-2-methoxyiminoacetamido]-3-[(E)-3-[(1,3,4-oxadiazol-2-yl)-methyldimethylammonium]-1-propen-1-yl]-3-cephem-4-carboxylat oder ein pharmakologisch annehmbares Salz davon ist.

6. Cephalosporin-Derivat oder dessen pharmakologisch annehmbares Salz gemäß Anspruch 1, dadurch **gekennzeichnet,** daß dieses Cephalosporin-Derivat oder dessen pharmakologisch annehmbares Salz 7β-[2-(5-Amino-1,2,4-thiadiazol-3-yl)-(Z)-2-methoxyiminoacetamido]-3-[(E)-3-(2-oxopropyldimethylammonium)-1-propen-1-yl]-3-cephem-4-carboxylat oder ein pharmakologisch annehmbares Salz davon ist.

7. Verfahren zur Herstellung eines Cephalosporin-Derivats, dargestellt durch die Formel:

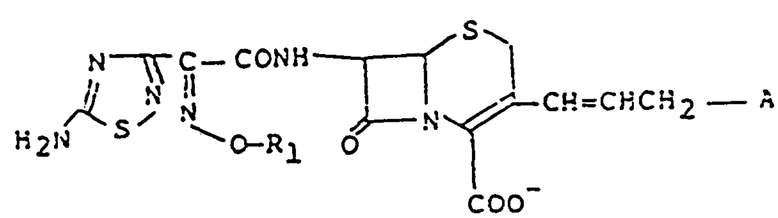

(I)

worin $R_1$ eine $C_{1-4}$-Alkyl-Gruppe ist und A ein quaternäres Ammoniumsalz, das A' entspricht, darstellt, oder von dessen pharmakologisch annehmbaren Salzen, das die Umsetzung einer Verbindung der Formel:

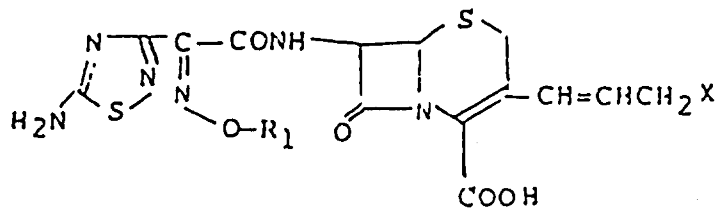 (II)

worin $R_1$ wie oben definiert ist und X ein Halogenatom ist, einer Verbindung, deren Amino- und/oder Carboxyl-Gruppe(n) durch eine Schutzgruppe geschützt sind, oder eines Salzes einer dieser Verbindungen mit einer Verbindung oder deren Salz, dargestellt durch die Formel:

A' (III)

umfaßt, wobei A' eine Gruppe der folgenden Formel ist:

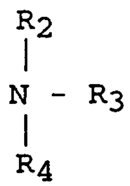

worin $R_2$ und $R_3$ gleiche oder verschiedene $C_{1-4}$-Alkyl-Gruppen sind, $R_4$ eine $C_{1-4}$-Alkyl-Gruppe ist, die mit einer Gruppe substituiert ist, die ausgewählt aus der Gruppe bestehend aus Carbamoyl-, Carboxyl-, $C_{1-4}$-Alkyl-substituierten-Amino-, Mercapto-, $C_{1-4}$-Alkanoyl-, Hydroxyl- oder Hydrazinocarbonyl-Gruppen; oder eine $C_{1-4}$-Alkyl-Gruppe ist, die mit einer 5-gliedrigen heterocyclischen Gruppe substituiert ist, welche als Substituenten haben kann: Amino, Carboxyl, $C_{1-4}$-Alkyl, Carboxy-$C_{1-4}$-alkyl, Carbamoyl, Hydroxyl, $C_{1-4}$-Alkyl-substituiertes Amino und Hydroxy-$C_{1-4}$-alkyl;
und gegebenenfalls eine Entfernung der Schutzgruppe umfaßt.

**8.** Antibakterielles Mittel, umfassend eine wirksame Menge eines Cephalosporin-Derivats, dargestellt durch die Formel

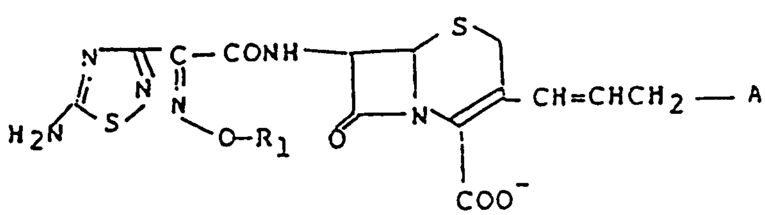 (I)

worin $R_1$ eine $C_{1-4}$-Alkyl-Gruppe darstellt und A eine Gruppe der folgenden Formel ist:

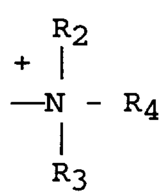

worin $R_2$ und $R_3$ gleiche oder verschiedene $C_{1-4}$-Alkyl-Gruppen sind, $R_4$ eine $C_{1-4}$-Alkyl-Gruppe ist, die substituiert ist mit einer Gruppe ausgewählt aus der Gruppe bestehend aus einer Carbamoyl-, Carboxyl-, $C_{1-4}$-alkyl-substituierten Amino-, Mercapto-, $C_{1-4}$-Alkanoyl-, Hydroxyl- oder Hydrazinocar-

bonyl-Gruppe; oder eine $C_{1-4}$-Alkyl-Gruppe ist, die mit einer 5-gliedrigen heterocyclischen Gruppe substituiert ist, welche als Substituenten haben kann: Amino, Carboxyl, $C_{1-4}$-Alkyl, Carboxy-$C_{1-4}$-alkyl, Carbamoyl, Hydroxyl, $C_{1-4}$-Alkyl-substituiertes Amino und eine Hydroxy-$C_{1-4}$-alkyl-Gruppe; oder dessen pharmakologisch annehmbares Salze; und einen pharmakologisch annehmbaren Träger.

**Patentansprüche für folgenden Vertragsstaat : AT**

**1.** Verfahren zur Herstellung eines Cephalosporin-Derivats, dargestellt durch die Formel:

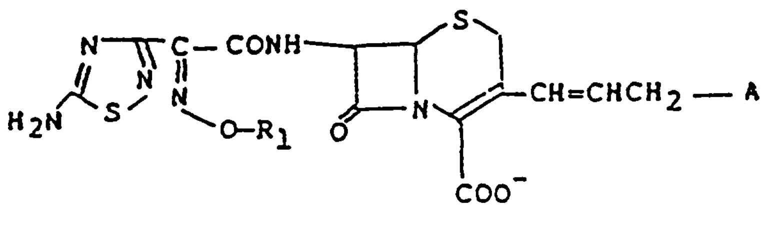

(I)

worin $R_1$ eine $C_{1-4}$-Alkyl-Gruppe ist und A ein quaternäres Ammoniumsalz darstellt, das A' entspricht, oder eines pharmakologisch annehmbaren Salzes davon, umfassend die Umsetzung einer Verbindung der Formel

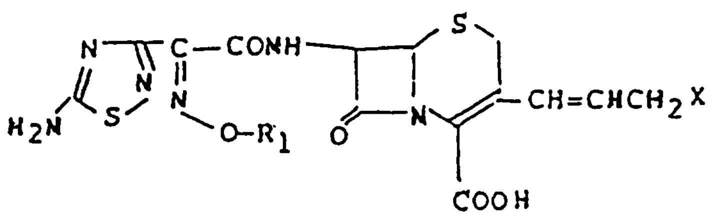

(II)

worin $R_1$ wie oben definiert ist und X ein Halogenatom ist, einer Verbindung, deren Amino- und/oder Carboxyl-Gruppe(n) durch eine Schutzgruppe geschützt sind, oder eines Salzes einer dieser Verbindungen, mit einer Verbindung der Formel:

A' (III)

oder einem Salz davon, wobei A' eine Gruppe der folgenden Formel ist:

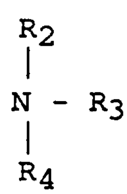

worin $R_2$ und $R_3$ gleiche oder verschiedene $C_{1-4}$-Alkyl-Gruppen sind, $R_4$ eine $C_{1-4}$-Alkyl-Gruppe ist, die mit einer Gruppe substituiert ist, die ausgewählt ist aus der Gruppe bestehend aus Carbamoyl-, Carboxyl-, $C_{1-4}$-Alkyl-substituierten-Amino-, Mercapto-, $C_{1-4}$-Alkanoyl-, Hydroxyl- oder Hydrazinocarbonyl-Gruppen; oder eine $C_{1-4}$-Alkyl-Gruppe ist, die mit einer 5-gliedrigen heterocyclischen Gruppe substituiert ist, welche als Substituenten haben kann: Amino-, Carboxyl-, $C_{1-4}$-Alkyl-, Carboxy-$C_{1-4}$-alkyl-, Carbamoyl-, Hydroxyl-, $C_{1-4}$-Alkyl-substituierte Amino- und Hydroxy-$C_{1-4}$-alkyl-Gruppen;
und gegebenenfalls Entfernung der Schutzgruppen.

**2.** Verfahren gemäß Anspruch 1, worin A in der Formel (I) eine Gruppe ist, die durch eine der folgenden Formeln darstellt wird:

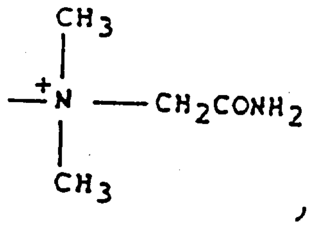 , 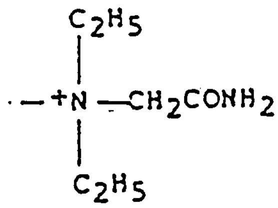 , 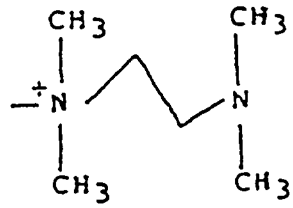 ,

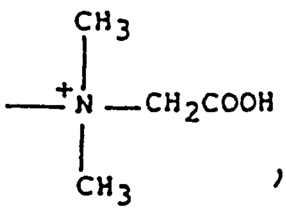 , 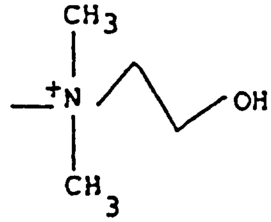 , 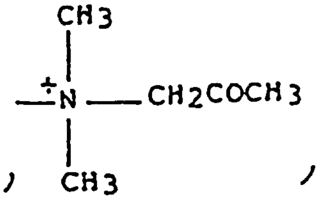 ,

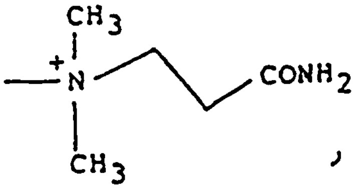 , 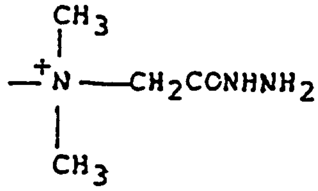 , 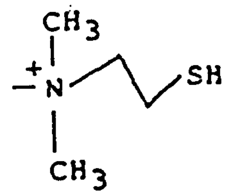 ,

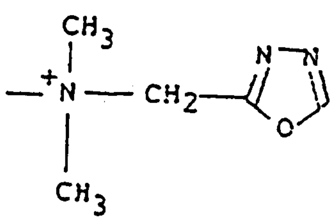 , 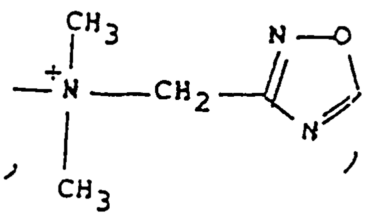 ,

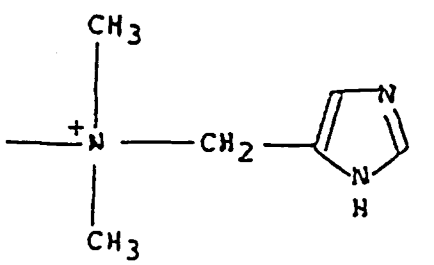 , 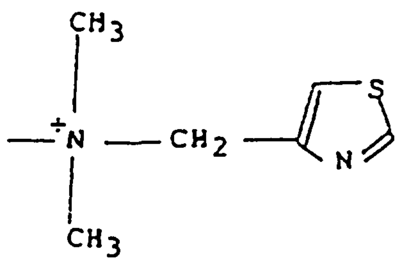 ,

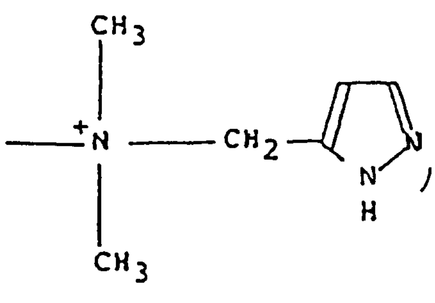 , 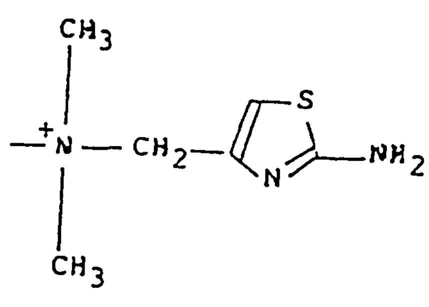 .

**3.** Verfahren gemäß Anspruch 1, dadurch **gekennzeichnet,** daß das Cephalosporin-Derivat oder dessen pharmakologisch annehmbares Salz 7b-[2-(5-Amino-1,2,4-thiadiazol-3-yl)-(Z)-2-methoxyiminoacetamido]-3-[(E)-3-(carbamoylmethyldimethylammonium)-1-propen-1-yl]-3-cephem-4-carboxylat oder ein pharmakologisch annehmbares Salz davon ist.

4. Verfahren gemäß Anspruch 1, dadurch **gekennzeichnet,** daß das Cephalosporin-Derivat oder dessen pharmakologisch annehmbares Salz 7b-[2-(5-Amino-1,2,4-thiadiazol-3-yl)-(Z)-2-methoxyiminoacetamido]-3-[(E)-3-(2-hydroxyethyldimethylammonium)-1-propen-1-yl]-3-cephem-4-carboxylat oder ein pharmakologisch annehmbares Salz davon ist.

5. Verfahren gemäß Anspruch 1, dadurch **gekennzeichnet,** daß das Cephalosporin-Derivat oder dessen pharmakologisch annehmbares Salz 7b-[2-(5-Amino-1,2,4-thiadiazol-3-yl)-(Z)-2-methoxyiminoacetamido]-3-[(E)-3-[(1,3,4-oxadiazol-2-yl)methyldimethylammonium]-1-propen-1-yl]-3-cephem-4-carboxylat oder ein pharmakologisch annehmbares Salz davon ist.

6. Verfahren gemäß Anspruch 1, dadurch **gekennzeichnet,** daß das Cephalosporin-Derivat oder dessen pharmakologisch annehmbares Salz 7b-[2-(5-Amino-1,2,4-thiadiazol-3-yl)-(Z)-2-methoxyiminoacetamido]-3-[(E)-3-(2-oxopropyldimethylammonium)-1-propen-1-yl]-3-cephem-4-carboxylat oder ein pharmakologisch annehmbares Salz davon ist.

7. Verwendung eines Cephalosporin-Derivats der Formel:

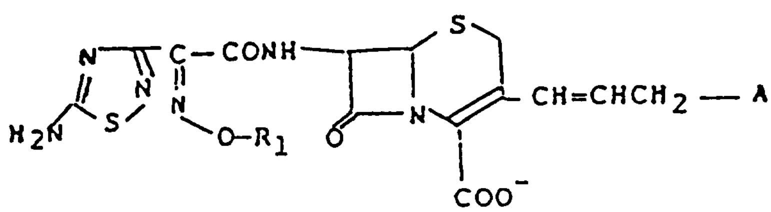

(I)

worin $R_1$ eine $C_{1-4}$-Alkyl-Gruppe darstellt und A eine Gruppe der folgenden Formel ist:

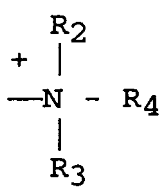

worin $R_2$ und $R_3$ gleiche oder verschiedene $C_{1-4}$-Alkyl-Gruppen sind, $R_4$ eine $C_{1-4}$-Alkyl-Gruppe ist, die mit einer Gruppe substituiert ist, die ausgewählt ist aus der Gruppe, bestehend aus Carbamoyl-, Carboxyl-, $C_{1-4}$-Alkyl-substituierten-Amino-, Mercapto-, $C_{1-4}$-Alkanoyl-, Hydroxyl- oder Hydrazinocarbonyl-Gruppen; oder eine $C_{1-4}$-Alkyl-Gruppe ist, die mit einer 5-gliedrigen heterocyclischen Gruppe substituiert ist, welche als Substituenten haben kann: Amino-, Carboxyl-, $C_{1-4}$-Alkyl-, Carboxy-$C_{1-4}$-alkyl-, Carbamoyl-, Hydroxyl-, $C_{1-4}$-Alkyl-substituierte Amino- und Hydroxy-$C_{1-4}$-alkyl-Gruppen; oder eines pharmakologisch annehmbaren Salzes davon
zur Herstellung eines antibakteriellen Mittels.

**Revendications**
**Revendications pour les Etats contractants suivants : BE, CH, DE, FR, GB, IT, LI, LU, NL, SE**

1. Dérivé de céphalosporine représenté par la formule :

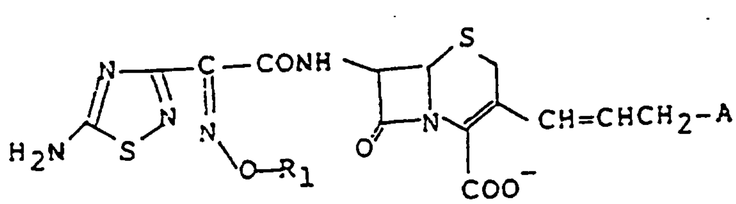

(I)

dans laquelle $R_1$ représente un groupe alkyle en $C_1$-$C_4$ et A est un groupe de formule suivante :

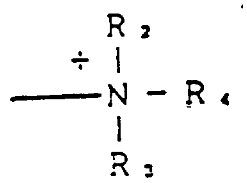

dans laquelle $R_2$ et $R_3$ sont un groupe alkyle en $C_1$-$C_4$ et sont identiques ou différents, $R_4$ est un groupe alkyle en $C_1$-$C_4$ substitué avec un groupe choisi parmi les groupes carbamoyle, carboxy, amino à substitution alkyle en $C_1$-$C_4$, mercapto, alcanoyle en $C_1$-$C_4$, hydroxy ou hydrazinocarbonyle ; un groupe alkyle en $C_1$-$C_4$ substitué avec un radical hétérocyclique à 5 chaînons qui peut avoir comme substituant un groupe amino, carboxy, alkyle en $C_1$-$C_4$, carboxy-alkyle en $C_1$-$C_4$, carbamoyle, hydroxy, amino à substitution alkyle en $C_1$-$C_4$ et hydroxyalkyle en $C_1$-$C_4$ ; ou un de ses sels pharmacologiquement acceptables.

**2.** Dérivé de céphalosporine ou un de ses sels pharmacologiquement acceptables selon la revendication 1, comprenant un composé ou un de ses sels pharmacologiquement acceptables où A dans la formule (I) est un groupe représenté par l'une des formules suivantes :

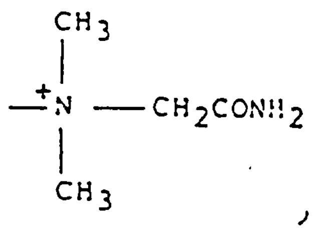 , 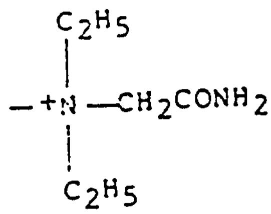 , 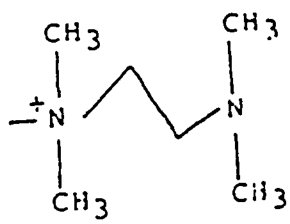 ,

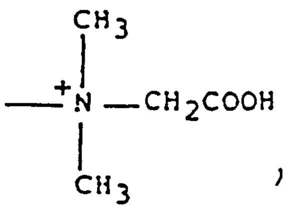 , 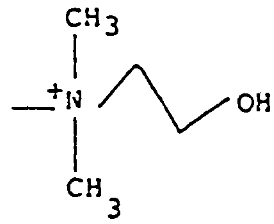 , 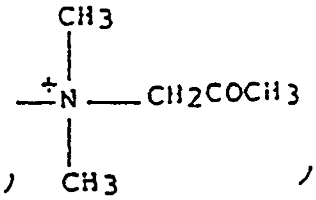 ,

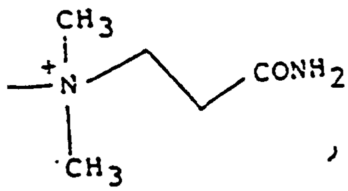 , 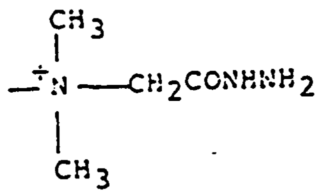 , 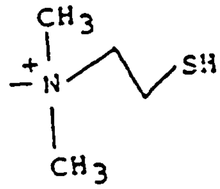 ,

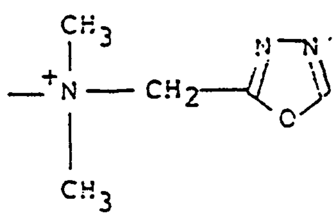 , 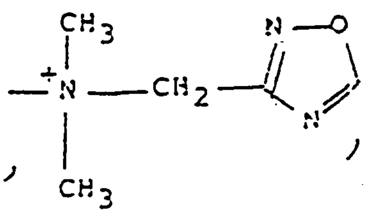 ,

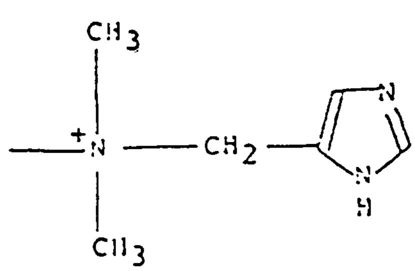 , 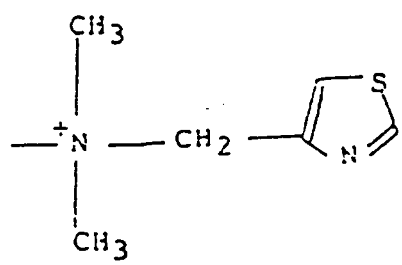 ,

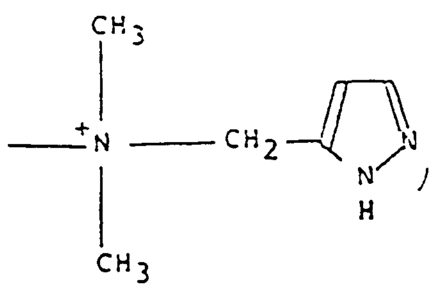 , 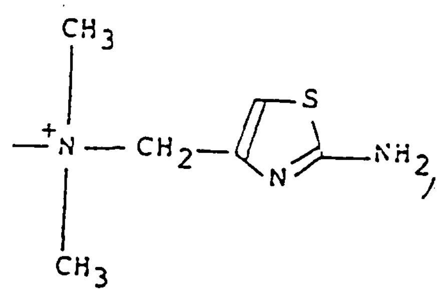 ,

3. Dérivé de céphalosporine ou un de ses sels pharmacologiquement acceptables selon la revendication 1, ledit dérivé de céphalosporine ou son sel pharmacologiquement acceptable étant le 7$\beta$-[2-(5-amino-1,2,4-thiadiazole-3-yl)-(Z)-2-méthoxyiminoacétamido]-3-[(E)-3-(carbamoylméthyldiméthylammonio)-1-propène-1-yl]-3-céphème-4-carboxylate ou un de ses sels pharmacologiquement acceptables.

4. Dérivé de céphalosporine ou un de ses sels pharmacologiquement acceptables selon la revendication 1, ledit dérivé de céphalosporine ou son sel pharmacologiquement acceptable étant le 7$\beta$-[2-(5-amino-1,2,4-thiadiazole-3-yl)-(Z)-2-méthoxyiminoacétamido]-3-[(E)-3-(2-hydroxyéthyldiméthylammonio)-1-propène-1-yl]-3-céphème-4-carboxylate ou un de ses sels pharmacologiquement acceptables.

**5.** Dérivé de céphalosporine ou un de ses sels pharmacologiquement acceptables selon la revendication 1, ledit dérivé de céphalosporine ou son sel pharmacologiquement acceptable étant le 7$\beta$-[2-(5-amino-1,2,4-thiadiazole-3-yl)-(Z)-2-méthoxyiminoacétamido]-3-[(E)-3-[(1,3,4-oxadiazole-2-yl)méthyldiméthyl-ammonio]-1-propène-1-yl]-3-céphème-4-carboxylate ou un de ses sels pharmacologiquement acceptables.

**6.** Dérivé de céphalosporine ou un de ses sels pharmacologiquement acceptables selon la revendication 1, ledit dérivé de céphalosporine ou son sel pharmacologiquement acceptable étant le 7$\beta$-[2-(5-amino-1,2,4-thiadiazole-3-yl)-(Z)-2-méthoxyiminoacétamido]-3-[(E)-3-(2-oxopropyldiméthylammonio)-1-propène-1-yl]-3-céphème-4-carboxylate ou un de ses sels pharmacologiquement acceptables.

**7.** Procédé pour préparer un dérivé de céphalosporine représenté par la formule :

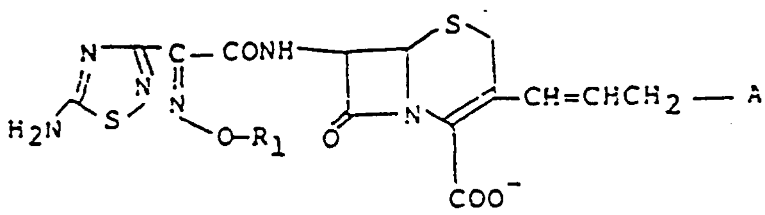

(I)

dans laquelle $R_1$ est un groupe alkyle en $C_1$-$C_4$ et A représente un sel d'ammonium quaternaire correspondant à A', ou un de ses sels pharmacologiquement acceptables, qui comprend la réaction d'un composé représenté par la formule :

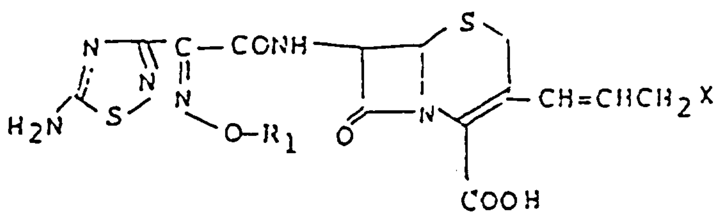

(II)

dans laquelle $R_1$ est tel que défini ci-dessus et X est un atome d'halogène, un tel composé dont le ou les groupes amino et/ou carboxy est/sont protégé(s) par un groupe protecteur, ou un sel d'un de ces composés, avec un composé ou un de ses sels représenté par la formule :

A' (III)

dans laquelle A' est un groupe de formule suivante :

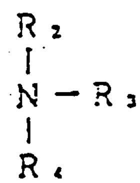

dans laquelle $R_2$ et $R_3$ sont un groupe alkyle en $C_1$-$C_4$ et sont identiques ou différents, $R_4$ est un groupe alkyle en $C_1$-$C_4$ substitué avec un groupe choisi parmi les groupes carbamoyle, carboxy, amino à substitution alkyle en $C_1$-$C_4$, mercapto, alcanoyle en $C_1$-$C_4$, hydroxy ou hydrazinocarbonyle ; un groupe alkyle en $C_1$-$C_4$ substitué avec un radical hétérocyclique à 5 chaînons qui peut avoir comme substituant un groupe amino, carboxy, alkyle en $C_1$-$C_4$, carboxy-alkyle en $C_1$-$C_4$, carbamoyle, hydroxy, amino à substitution alkyle en $C_1$-$C_4$ et hydroxyalkyle en $C_1$-$C_4$ ;
et facultativement l'élimination du groupe protecteur.

**8.** Agent antibactérien comprenant :

une quantité efficace d'un dérivé de céphalosporine représenté par la formule :

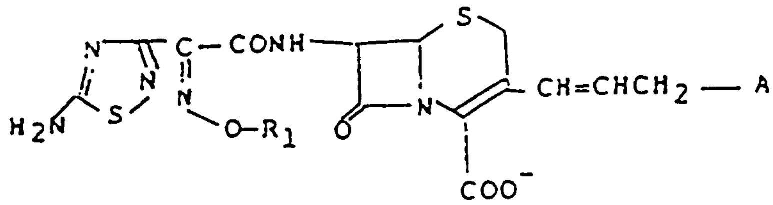 (I)

dans laquelle $R_1$ représente un groupe alkyle en $C_1$-$C_4$ et A est un groupe de formule suivante :

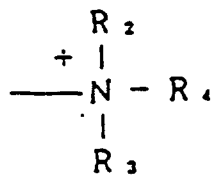

dans laquelle $R_2$ et $R_3$ sont un groupe alkyle en $C_1$-$C_4$ et sont identiques ou différents, $R_4$ est un groupe alkyle en $C_1$-$C_4$ substitué avec un groupe choisi parmi les groupes carbamoyle, carboxy, amino à substitution alkyle en $C_1$-$C_4$, mercapto, alcanoyle en $C_1$-$C_4$, hydroxy ou hydrazinocarbonyle ; un groupe alkyle en $C_1$-$C_4$ substitué avec un radical hétérocyclique à 5 chaînons qui peut avoir comme substituant un groupe amino, carboxy, alkyle en $C_1$-$C_4$, carboxy-alkyle en $C_1$-$C_4$, carbamoyle, hydroxy, amino à substitution alkyle en $C_1$-$C_4$ et hydroxyalkyle en $C_1$-$C_4$ ; ou un de ses sels pharmacologiquement acceptables ; et un excipient pharmacologiquement acceptable.

**Revendications pour l'Etat contractant suivant : AT**

**1.** Procédé pour préparer un dérivé de céphalosporine représenté par la formule :

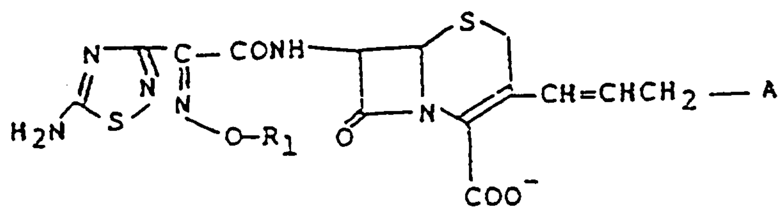 (I)

dans laquelle $R_1$ est un groupe alkyle en $C_1$-$C_4$ et A représente un sel d'ammonium quaternaire correspondant à A', ou un de ses sels pharmacologiquement acceptables, qui comprend la réaction d'un composé représenté par la formule :

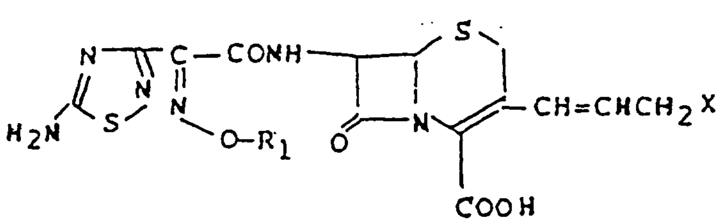 (II)

dans laquelle $R_1$ est tel que défini ci-dessus et X est un atome d'halogène, un tel composé dont le ou les groupes amino et/ou carboxy est/sont protégé(s) par un groupe protecteur, ou un sel d'un de ces composés, avec un composé ou un de ses sels représenté par la formule :

A' (III)

dans laquelle A' est un groupe de formule suivante :

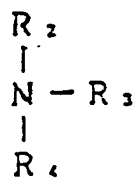

dans laquelle $R_2$ et $R_3$ sont un groupe alkyle en $C_1$-$C_4$ et sont identiques ou différents, $R_4$ est un groupe alkyle en $C_1$-$C_4$ substitué avec un groupe choisi parmi les groupes carbamoyle, carboxy, amino à substitution alkyle en $C_1$-$C_4$, mercapto, alcanoyle en $C_1$-$C_4$, hydroxy ou hydrazinocarbonyle ; un groupe alkyle en $C_1$-$C_4$ substitué avec un radical hétérocyclique à 5 chaînons qui peut avoir comme substituant un groupe amino, carboxy, alkyle en $C_1$-$C_4$, carboxy-alkyle en $C_1$-$C_4$, carbamoyle, hydroxy, amino à substitution alkyle en $C_1$-$C_4$ et hydroxyalkyle en $C_1$-$C_4$ ;
et facultativement l'élimination du groupe protecteur.

**2.** Procédé selon la revendication 1, où A dans la formule (I) est un groupe représenté par l'une des formules suivantes :

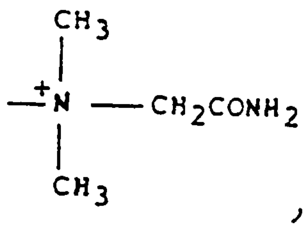,
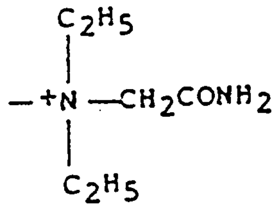,
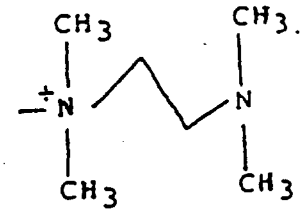,

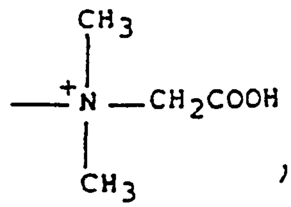,
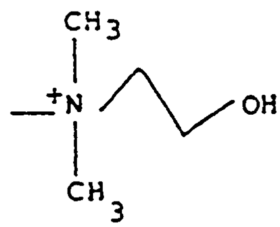,
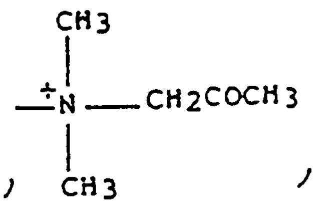,

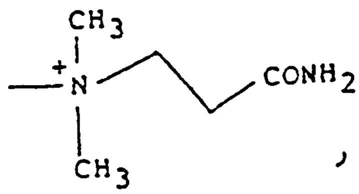,
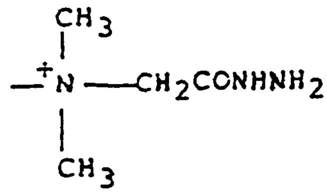,
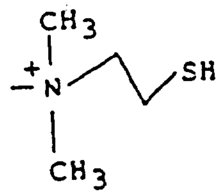,

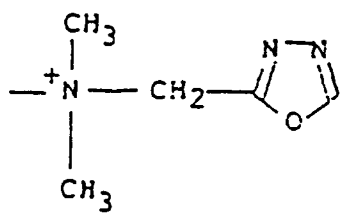,
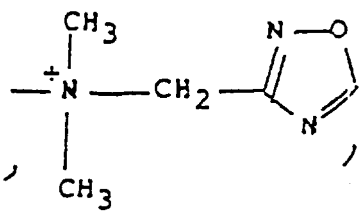,

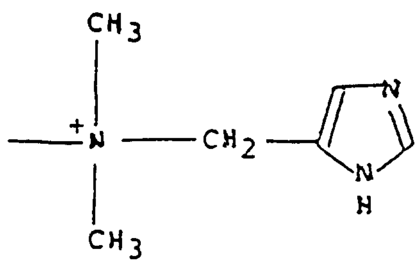,
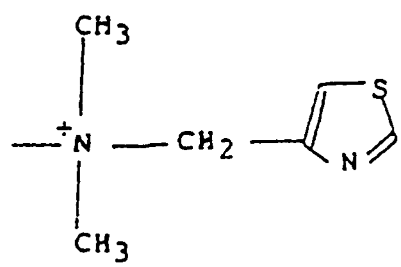,

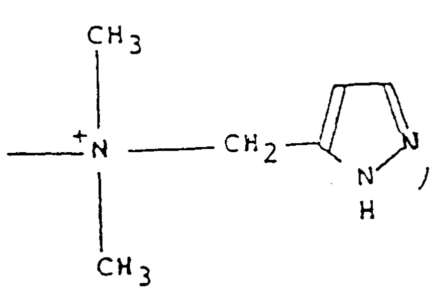,
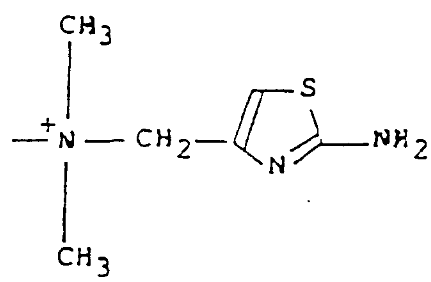.

3. Procédé selon la revendication 1, où ledit dérivé de céphalosporine ou son sel pharmacologiquement acceptable est le 7β-[2-(5-amino-1,2,4-thiadiazole-3-yl)-(Z)-2-méthoxyiminoacétamido]-3-[(E)-3-(carbamoylméthyldiméthylammonio)-1-propène-1-yl]-3-céphème-4-carboxylate ou un de ses sels pharmacologiquement acceptables.

4. Procédé selon la revendication 1, où ledit dérivé de céphalosporine ou son sel pharmacologiquement acceptable est le 7β-[2-(5-amino-1,2,4-thiadiazole-3-yl)-(Z)-2-méthoxyiminoacétamido]-3-[(E)-3-(2-hydroxyéthyldiméthylammonio)-1-propène-1-yl]-3-céphème-4-carboxylate ou un de ses sels pharmacologiquement acceptables.

5. Procédé selon la revendication 1, où ledit dérivé de céphalosporine ou son sel pharmacologiquement acceptable est le 7β-[2-(5-amino-1,2,4-thiadiazole-3-yl)-(Z)-2-méthoxyiminoacétamido]-3-[(E)-3-[(1,3,4-oxadiazole-2-yl)méthyldiméthylammonio]-1-propène-1-yl]-3-céphème-4-carboxylate ou un de ses sels pharmacologiquement acceptables.

6. Procédé selon la revendication 1, où ledit dérivé de céphalosporine ou son sel pharmacologiquement acceptable est le 7β-[2-(5-amino-1,2,4-thiadiazole-3-yl)-(Z)-2-méthoxyiminoacétamido]-3-[(E)-3-(2-oxopropyldiméthylammonio)-1-propène-1-yl]-3-céphème-4-carboxylate ou un de ses sels pharmacologiquement acceptables.

7. Utilisation d'un dérivé de céphalosporine représenté par la formule :

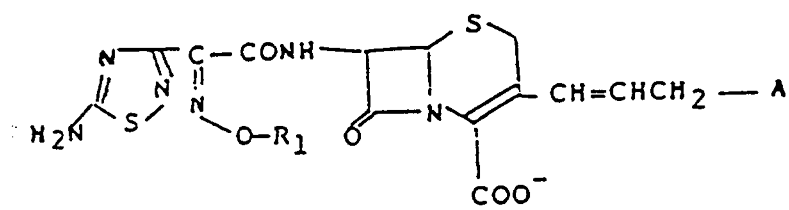

(I)

dans laquelle $R_1$ représente un groupe alkyle en $C_1$-$C_4$ et A est un groupe de formule suivante :

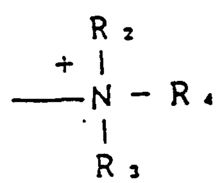

dans laquelle $R_2$ et $R_3$ sont un groupe alkyle en $C_1$-$C_4$ et sont identiques ou différents, $R_4$ est un groupe alkyle en $C_1$-$C_4$ substitué avec un groupe choisi parmi les groupes carbamoyle, carboxy, amino à substitution alkyle en $C_1$-$C_4$, mercapto, alcanoyle en $C_1$-$C_4$, hydroxy ou hydrazinocarbonyle ; un groupe alkyle en $C_1$-$C_4$ substitué avec un radical hétérocyclique à 5 chaînons qui peut avoir comme substituant un groupe amino, carboxy, alkyle en $C_1$-$C_4$, carboxy-alkyle en $C_1$-$C_4$, carbamoyle, hydroxy, amino à substitution alkyle en $C_1$-$C_4$ et hydroxyalkyle en $C_1$-$C_4$ ; ou d'un de ses sels pharmacologiquement acceptables ; pour la préparation d'un agent antibactérien.